(19) Europäisches Patentamt
European Patent Office
Office européen des brevets

(11) EP 4 772 860 A1

(12) EUROPEAN PATENT APPLICATION

(43) Date of publication:
08.07.2026 Bulletin 2026/28

(51) International Patent Classification (IPC):
G01N 21/3577 (2014.01)   G01N 33/14 (2006.01)
G01N 21/359 (2014.01)   G01N 21/85 (2006.01)

(21) Application number: 26150548.1

(22) Date of filing: 07.01.2026

(52) Cooperative Patent Classification (CPC):
G01N 21/3577; G01N 21/359; G01N 21/85;
G01N 33/14; G01N 33/146; G01N 21/0332

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 07.01.2025 US 202563742734 P

(71) Applicant: Haffmans B.V.
5928 PW Venlo (NL)

(72) Inventors:
• LEVELS, Geert
5928 PW Venlo (NL)
• JANS, Theo
5928 PW Venlo (NL)
• Van KEMPEN, Frank
5928 PW Venlo (NL)
• GÜNTHER, Christian Wilhelm
5928 PW Venlo (NL)

(74) Representative: Viering, Jentschura & Partner
mbB
Patent- und Rechtsanwälte
Am Brauhaus 8
01099 Dresden (DE)

(54) **SYSTEMS AND METHODS FOR OPTICALLY DETERMINING THE CONCENTRATION OF ALCOHOL AND EXTRACT IN A LIQUID SAMPLE**

(57) An apparatus designed to determine one or more parameters of a fluid sample includes a housing, a fluid conduit, a light source, a measurement cell, a detector, one or more heat exchangers, and a density sensor. The apparatus imparts the fluid sample with a target temperature. The housing includes an inlet and an outlet, with the fluid conduit disposed in the housing and defining a fluid flow path between the inlet and the outlet. The measurement cell is in optical communication with the light source, and the detector determines an absorption spectrum of the fluid sample. The one or more heat exchangers include a first heat exchanger and a second heat exchanger, where the first heat exchanger and the second heat exchanger are arranged in the fluid flow path and are in thermal communication with the fluid conduit. The density sensor measures a density of the fluid sample.

FIG. 2

## Description

CROSS-REFERENCE TO RELATED APPLICATIONS

This application claims priority to U.S. Provisional Patent Application Serial No. 63/742,734, filed on January 7, 2025, entitled "SYSTEM AND METHOD FOR OPTICALLY DETERMINING THE CONCENTRATION OF ALCOHOL AND EXTRACT IN A LIQUID SAMPLE," currently pending, the entire disclosure of which is incorporated herein by reference.

FIELD OF DISCLOSURE

The present disclosure relates to systems and methods for optically determining the concentration of compounds in a liquid sample. More particularly, the present disclosure relates to systems and methods for determining the concentration of components such as alcohol and extracts in beverage samples using near-infrared spectroscopy.

BACKGROUND

Beer and other alcoholic beverages are produced and consumed worldwide. Because of the large consumer market for alcoholic beverages, and beer in particular, there has always been great interest in better controlling the production process of beer. Early instruments, such as the thermometer and hydrometer, allowed brewers to measure variables such as temperature and extract concentration, which in turn provided the brewers information about the brewing process and the products thereof.

Today, there are several methods for determining the alcohol content of beers, including FTIR spectroscopy, fluorescent spectroscopy, gas chromatography, high-performance liquid chromatography, distillation, ebuliometry, and chemical methods such as enzymatic determination and dichromatic oxidation. In general, the techniques for determining analyte content in beer suffer from various shortcomings. Some of those shortcomings include requiring separate, distinct test procedures for the different analytes found in the same liquid, the use of specialized equipment, and/or requiring personnel knowledgeable about the scientific principles underlying the procedure. Each of these requirements represent a significant investment in time and cost for the brewers and often renders these techniques unaffordable for smaller brewers.

While near-infrared spectrometry ("NIR") is often touted as a solution to these problems, solely relying on NIR introduces its own obstacles. Specifically, NIR measurements are typically highly temperature dependent, making it difficult to identify and determine the concentration of the components of a sample if the temperature of the sample is not controlled. To combat this, some NIR manufacturers recommend only collecting data for the very limited portions of the absorption spectra that are not temperature dependent. However, this reduces the ability of the NIR instrument to identify the various components that may be found in a sample.

Thus, there is a need for improved systems and methods for accurately determining the concentration of the components of alcoholic beverages, including beer.

SUMMARY

In one aspect, an apparatus designed to determine one or more parameters of a fluid sample is provided in the form of a housing, a fluid conduit, a light source, a measurement cell, a detector, one or more heat exchangers, and a density sensor. The apparatus is designed to impart the fluid sample with a target temperature. The housing includes an inlet and an outlet, with the fluid conduit disposed in the housing and defining a fluid flow path between the inlet and the outlet. The measurement cell is in optical communication with the light source and the detector is designed to determine an absorption spectrum of the fluid sample. The one or more heat exchangers include a first heat exchanger and a second heat exchanger, where the first heat exchanger and the second heat exchanger are arranged in the fluid flow path and are in thermal communication with the fluid conduit. The density sensor is designed to measure a density of the fluid sample.

In some cases, the first heat exchanger and the second heat exchanger are arranged in series, and the first heat exchanger is positioned upstream of the second heat exchanger relative to a flow of the fluid sample through the fluid flow path.

In some instances, the first heat exchanger is designed to impart the fluid sample with a first temperature within about 1 °C to about 10 °C of the target temperature, and the second heat exchanger is designed to impart the fluid sample with a second temperature within about 0 °C to about 1 °C of the target temperature.

In various cases, the apparatus further includes a third heat exchanger arranged in series with the first heat exchanger and the second heat exchanger. The third heat exchanger is positioned downstream of the second heat exchanger relative to a flow of the fluid sample through the fluid flow path, and the third heat exchanger is designed to impart the fluid sample with a third temperature within about 0.01 °C of the target temperature.

In some instances, the detector is provided as part of a NIR spectrometer system.

In some cases, the apparatus further includes a controller in electronic communication with the detector and the density sensor. The controller analyzes measurements provided from or received from the detector and the density sensor to generate a PLS regression model, and the controller determines if the fluid sample is an authentic beverage upon comparison to a known beverage profile.

In some instances, the one or more heat exchangers are designed to impart the fluid sample with a temperature of at least 10 °C before the fluid sample is provided to the measurement cell.

In various instances, the one or more heat exchangers are designed to impart the fluid sample with a temperature of about 20 °C to about 25 °C before the absorption spectrum is determined by the detector.

In various cases, the fluid sample is imparted with a first temperature, and the one or more heat exchangers are designed to impart the fluid sample with a second temperature that is within a predetermined tolerance of the target temperature. In addition, the fluid sample is imparted with the second temperature before the fluid sample is provided to the measurement cell.

In some instances, the apparatus measures one or more parameters of the fluid sample, and the one or more parameters include one or more of a concentration of alcohol, a pH level, a sugar content, a flow rate, a dissolved carbon dioxide concentration, a concentration of one or more aromatics, a protein content, a bitterness value, or a temperature of the fluid sample.

In some aspects, the techniques described herein relate to a system, including a beverage manufacturing line and a measurement device. The measurement device is in fluid communication with the beverage manufacturing line. The measurement device includes a measurement cell designed to receive a fluid sample, where the fluid sample is imparted with a first concentration of extract and a second concentration of alcohol, one or more heat exchangers, an NIR spectrometer including a detector positioned to receive light that passes through the measurement cell, and a controller. The controller is in communication with the one or more heat exchangers and the NIR spectrometer.

In some instances, the measurement device is positioned in-line with the beverage manufacturing line.

In various cases, the controller is housed within the measurement device, the measurement device provides measurements of one or more parameters to the controller, and the controller determines a PLS regression model utilizing the measurements.

In various instances, the controller determines a third concentration of a first component of the fluid sample, and the third concentration is selected from the group consisting of an alcohol content, a sugar content, an extract concentration, a total carbohydrate content, a dissolved carbon dioxide concentration, a concentration of aromatics, a protein content, and an IBU value.

In some cases, a temperature of the fluid sample is controlled before being provided to the NIR spectrometer when the measurement device is positioned at-line in the beverage manufacturing line. Alternatively, the controller utilizes a PLS regression model to compensate for an effect of the temperature of the fluid sample when the measurement device is positioned in-line or on-line in the beverage manufacturing line.

In some aspects, a method for determining concentrations of one or more constituents of a beverage is provided. The method includes: providing the beverage, where the beverage includes one or more constituents; providing a sample of the beverage to an apparatus including a detector and a density sensor; controlling a temperature of the beverage with one or more heat exchangers associated with the apparatus; measuring a density of the beverage using the density sensor; and determining a concentration of a first constituent of the one or more constituents of the beverage using the detector.

In some cases, a controller determines the concentration of the first constituent at least partially based on an absorption spectrum determined or obtained by the apparatus.

In some instances, the method further includes determining an identity of the beverage utilizing an absorption spectrum created from data obtained from the apparatus.

In various instances, the first constituent is selected from the group consisting of alcohol, sugar, extract, carbon dioxide, a total carbohydrate content, aromatics, proteins, and bitterness-imparting compounds.

In some cases, the first constituent is alcohol or extract.

DESCRIPTION OF THE DRAWINGS

FIG. 1A is a schematic diagram depicting a system for optically determining a concentration of beverage components, the system including a light splitter;
FIG. 1B is a schematic diagram depicting a system for optically determining a concentration of beverage components, in which a light splitter is omitted from the system;
FIG. 2 is an isometric view of a system for optically determining beverage component concentration;

FIG. 3 is a schematic block diagram of a control system that can be utilized with the systems of FIGS. 1A, 1B, and 2;

FIG. 4 is an isometric view of the system of FIG. 2 with an outer housing removed to illustrate various internal components of the system;

FIG. 5 is a cross-sectional view of the various internal components of the system of FIG. 4, the cross-section taken along line 5-5 of FIG. 4;

FIG. 6 is an isometric view of the system of FIG. 2 with the outer housing removed to illustrate an alternative arrangement of various internal components of the system;

FIG. 7 is a cross-sectional view of the various internal components of the system of FIG. 6, the cross-section taken along line 7-7 of FIG. 6;

FIG. 8 is a schematic diagram depicting a system for optically determining concentrations of beverage components that is arranged in-line with various beverage manufacturing machines; and

FIG. 9 is a block diagram illustrating a method for determining the concentrations of constituents of a beverage sample.

These and other aspects and advantages of the present disclosure will become apparent to those skilled in the art after considering the following detailed description in connection with the accompanying drawings.

DETAILED DESCRIPTION

Before any instances of the disclosure are explained in detail, it is to be understood that the disclosure is not limited in its application to the details of construction and the arrangement of components set forth in the following description or illustrated in the following drawings. The disclosure is capable of other instances and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The following discussion is presented to enable a person skilled in the art to make and use instances of the disclosure. Various modifications to the illustrated instances will be readily apparent to those skilled in the art, and the generic principles herein can be applied to other instances and applications without departing from instances of the disclosure. Thus, instances of the disclosure are not intended to be limited to instances shown but are to be accorded the widest scope consistent with the principles and features disclosed herein. The following detailed description is to be read with reference to the figures, in which like elements in different figures have like reference numerals. The figures, which are not necessarily to scale, depict selected instances and are not intended to limit the scope of instances of the disclosure. Skilled artisans will recognize the examples provided herein have many useful alternatives and fall within the scope of instances of the disclosure.

According to the teachings herein, apparatuses and methods for determining concentrations of selected components, substances, compounds, or constituents of a beverage sample are provided. The beverage samples analyzed by the apparatuses may include, but are not limited to, beer, wine, spirits, and non-alcoholic beverages. The selected components, substances, compounds, or constituents can include, but are not limited to, alcohol, sugars, other carbohydrates, proteins, and aromatics. As will be described herein, the apparatuses may determine the concentrations of alcohol and extract in the beverage sample. In certain instances, and in conjunction with other instruments such as Nuclear Magnetic Resonance ("NMR") spectrometers and density measurement devices, the apparatuses can determine the identity and concentration of components selected from the group consisting of an alcohol, a sugar, carbon dioxide, an aromatic, a protein, and combinations thereof.

In some cases, the apparatuses disclosed herein may be used to determine an identity and/or a concentration of one or more compounds of a beverage sample. For instance, the apparatuses disclosed herein may be used to aid in fraud detection. As beverages are created, the beverages are imparted with predetermined concentrations of various substances including, but not limited to, alcohol, sugars, dissolved carbon dioxide, aromatics and proteins. Using the apparatuses described herein, an unknown sample can be compared to a "fingerprint" of the beverage sample to determine whether the unknown sample is authentic.

In certain instances, the aromatics identified by the apparatuses disclosed herein may include esters, volatile alcohols, aldehydes, phenols, terpenes, volatile acids, and other scent-imparting compounds found in alcoholic beverages (e.g., beer, cider, wine). In certain cases, the alcohol content detected by the apparatus may be an ethanol content of the alcoholic beverage. In various instances, the bitterness-imparting compounds provided in the beverage may be iso-alpha acids. In such instances, the concentration of the bitterness may be measured in International Bitterness Units (IBUs).

In certain cases, the apparatuses described herein may also be used to determine the quality of beverages produced in a production line. For example, the apparatuses may be used to determine if the ingredients of a beverage sample are imparted with concentration values (e.g., an extract concentration) that are within a defined tolerance or threshold. As an additional example, the apparatuses may be used to determine if the beverage samples produced by the beverage line are imparted with ingredient concentrations that do not fall outside of a defined tolerance over time. If it is determined that the ingredient concentrations fall outside of the defined tolerance, then the beverage manufacturer can adjust the manufacturing process to bring the ingredient concentrations back into the defined tolerances.

The apparatuses described herein may also determine the concentration and identification of compounds in a beverage sample using near-infrared ("NIR") spectroscopy and various mathematical models (e.g., a partial least squares regression ("PLS") model). The PLS regression model restructures the NIR spectral data obtained from the apparatuses based on the spectral data's covariance with chosen response variables. In other words, the PLS regression model isolates wavelengths in the obtained spectra whose intensity values are most strongly correlated to the alcohol or extract content (e.g., carbohydrates, proteins) of the beverage samples. The original variables of the dataset are combined to form a new set of variables, referred to as latent variables or "LVs." These combined variables are meant to be more informative than the original, single variables because the combined variables represent larger trends in the original data, e.g., patterns of peaks and other features which are present at multiple points in the spectrum. Thus, prediction models can typically be built using a small number of LVs.

As used in the apparatuses described herein, NIR spectroscopy may also be used to authenticate or "fingerprint" beverages. In some instances, a "fingerprint" of a particular beverage may delineate the compounds and the concentrations thereof of a sample, and the fingerprint may then be compared to a fingerprint of a known sample. Generally, NIR spectroscopy is a vibrational spectroscopy technique in which electromagnetic radiation probes molecular vibrations. NIR spectroscopy provides fast, non-destructive, and cost-effective measurements, and oftentimes does not require sample preparation. These properties make NIR techniques suitable for food products, including alcoholic beverages, (e.g., beer).

The fingerprints of the known samples can be determined in a variety of manners. For example, the fingerprint of the known sample may be generated via analysis of data obtained via NIR spectrometry. Then, by employing Data-Driven Soft Independent Modeling Technology (DD-SIMCA), the spectra of the unknown samples may be compared to the spectra of an initial calibration set, i.e., the known sample, to determine the unknown sample's authenticity. As such, NIR spectroscopy can help determine possible adulteration or fraud of known beverages. In certain cases, NMR analysis may also be carried out to determine a concentration of alcohol and other components in the sample. The data obtained from the NMR analysis corresponds to the determined NMR absorption spectrum.

In some instances, the disclosed apparatuses may be used to determine the extract concentration and/or total sugar concentration of a beverage sample. The determination can be made using NIR spectroscopy with absolute reference data. As such, since the total concentration of carbohydrates (alcohol and sugars) can be determined in a sample, the caloric content of the sample can also be determined.

In some cases, the apparatuses described herein may be used in an in-line configuration. In such instances, the apparatuses may be arranged in the beverage line's process flow, and the apparatuses may make measurements of the process flow continuously or at defined time intervals. In other cases, the apparatuses may be provided in an at-line configuration, in which a sample is removed from the beverage line for analysis (e.g., at a quality control station) and then either disposed of or returned to the beverage line. In yet other cases, the apparatuses may be used in an on-line configuration. In such cases, the apparatuses may be in fluid communication with the process flow, but a sample is diverted from the main process flow of the beverage line and provided to the apparatus for measurement. After the measurement is complete, the sample may be returned to the manufacturing line.

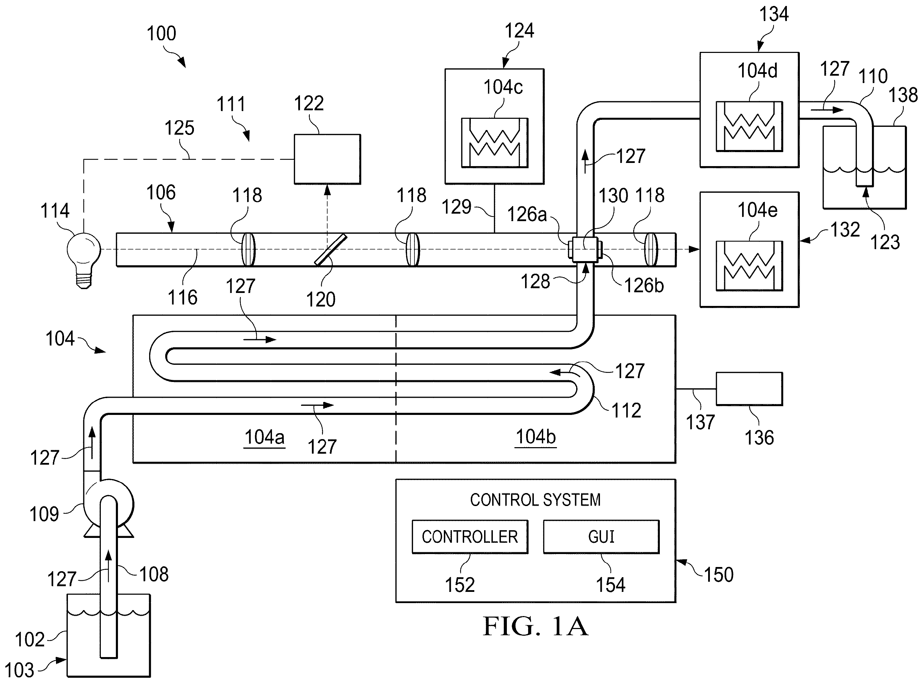

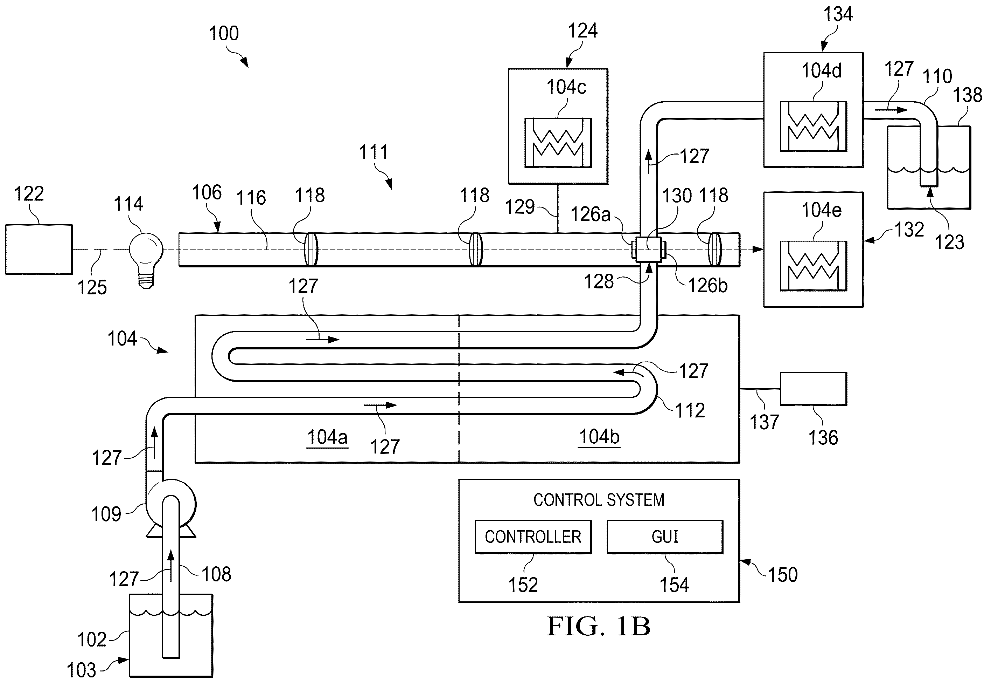

Referring now to FIGS. 1A and 1B, a schematic illustration of measurement components, temperature control elements, and other components of an apparatus 100 is provided. The apparatus 100 may be configured or designed to measure a parameter or a quality of a fluid sample (e.g., beer, cider, wine, liquor, non-alcoholic beverages, etc.). In some instances, the apparatus 100 may include a sample source 102 in fluid communication with a beverage sample 103, one or more heat exchangers 104 (e.g., a first heat exchanger 104a, a second heat exchanger 104b, a third heat exchanger 104c, a fourth heat exchanger 104d, a fifth heat exchanger 104e), a pump 109, an NIR spectrometer system 111, and a density sensor 134. To measure one or more parameters of the beverage sample 103, the apparatus 100 may extract the beverage sample 103 from the sample source 102 through an input conduit 108 operatively connected to the pump 109. The input conduit 108 may be coupled to one or more fluid conduits (e.g., a fluid conduit 112) provided within the apparatus 100 (e.g., within the first and second heat exchangers 104a, 104b).

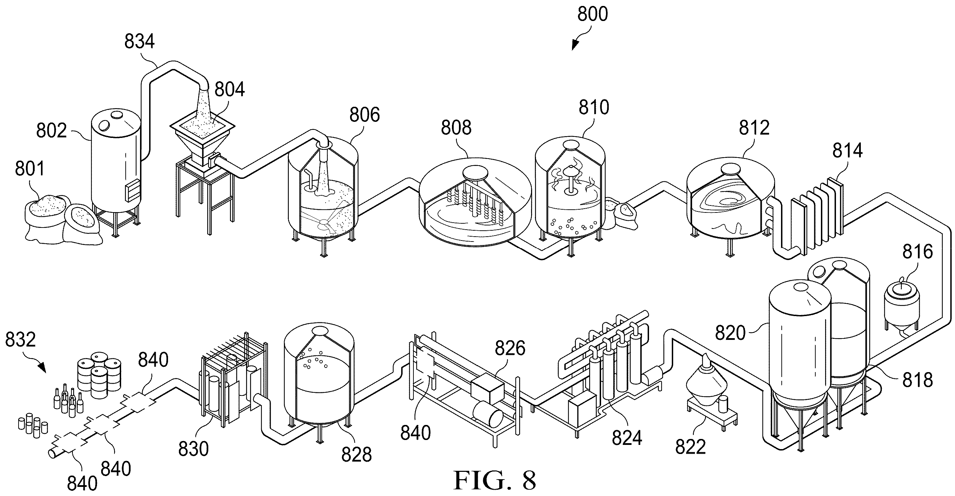

In various instances, the sample source 102 may be a beverage production line, such as a beverage manufacturing system 800 described with reference to FIG. 8, although the sample source 102 may be provided in other forms. The apparatus 100 may be used to analyze a variety of liquid samples. As such, the beverage sample 103 may be provided in the form of beer, cider, wine, liquor, sparkling juices, non-alcoholic beverages, and/or other similar beverages.

Referring again to FIGS. 1A and 1B, the one or more heat exchangers 104 may help control the temperature of the beverage sample 103, or components of the apparatus 100, as the beverage sample 103 is conveyed through the apparatus 100 prior to measurement. As will be further described herein, by actively heating or cooling the beverage sample 103 (and/or the components used to measure various parameters of the beverage sample 103) to a predetermined target temperature or within a defined tolerance thereof, the one or more heat exchangers 104 may help ensure that the sample is measured under stable and controlled thermal conditions. This temperature regulation may help improve the accuracy and reliability of optical measurements, such as those performed by the NIR spectrometer system 111 and the density sensor 134, which may be sensitive to temperature fluctuations. By minimizing temperature-induced variability, the one or more heat exchangers 104 may help provide more consistent, reproducible, and accurate determination of analyte concentrations (e.g., alcohol and extract) in the sample. In certain instances, multiple heat exchangers may be arranged in series, with each stage progressively bringing the sample closer to the predetermined or target temperature, thereby helping to facilitate fine-tuned thermal control and improved measurement performance. In some cases, each heat exchanger of the one or more heat exchangers 104 may be provided with a heat sink. In other cases, at least two of the heat exchangers of the one or more heat exchangers 104 may share a single heat sink.

The NIR spectrometer system 111 may be substantially similar to the NIR spectrometer systems described above. In various instances, the NIR spectrometer system 111 may be configured or designed to determine a parameter of a beverage (e.g., a concentration of one or more components of the beverage). The NIR spectrometer system 111 may include an optical device 106 provided in the form of a light source 114, one or more lenses 118, a splitter 120 (which may be omitted, as in FIG. 1B), a light control mechanism 122, an optics heat control device 124, and a measurement cell 128. The light source 114 may be configured or designed to provide light along an optical light path 116 within the optical device 106. The NIR spectrometer system 111 may also include the detector 132. The detector 132 may be configured or designed to measure characteristics of light emitted by the light source 114 after the light passes through the measurement cell 128. In certain cases, the light source 114 may provide light through the optical device 106 and to the light control mechanism 122. The light control mechanism 122 may be connected to the light source 114 via a first connection 125. As such, in some instances, the light control mechanism 122 may monitor the light produced by the light source 114 and adjust the output of the light source 114 as needed.

In some instances, a temperature of the optical device 106 may be regulated by an optics heat control device 124. The optics heat control device 124 may be connected to the optical device 106 by a second connection 129. The optics heat control device 124 may include the third heat exchanger 104c.

Still referring to FIGS. 1A and 1B, the measurement cell 128 may include a chamber 130 comprising stainless steel, although other durable, non-reactive materials may also be used in the construction of the chamber 130. The chamber 130 may include a first window 126a and a second window 126b that may be positioned on opposite sides of the chamber 130. The first window 126a and the second window 126b may be configured or designed to direct light in the optical light path 116 into and out of the measurement cell 128. Stated another way, the first and second windows 126a, 126b may allow for light to enter and exit the measurement cell 128. In addition, the chamber 130 can be positioned in the NIR spectrometer system 111 such that the light generated by the light source 114 can pass through the first window 126a, the chamber 130, and the second window 126b and eventually to the detector 132.

The density sensor 134 may be in fluid communication with the NIR spectrometer system 111. For example, the density sensor 134 may be in fluid communication with, and downstream of, the measurement cell 128. The density sensor 134 may measure the density of the beverage sample 103 as the beverage sample 103 passes through the apparatus 100. Thus, the density sensor 134 may help determine physical properties of the beverage sample 103, which can be used independently or in combination with optical measurements (such as those obtained via the NIR spectrometer system 111) to calculate the concentrations of various components of the beverage sample 103, including alcohol and extract. Accurate density measurement may be important in beverage analysis, as density is directly correlated to parameters such as alcohol content, sugar concentration, and extract levels. The density sensor 134 may thus help enhance the reliability and accuracy of the overall analytical process associated with the apparatus 100, may help cross-validate results obtained from the NIR spectrometer system 111, and may help identify and authenticate the beverage sample 103. In certain instances, the density sensor 134 may also provide additional parameters, such as a specific gravity of the beverage sample 103, further supporting comprehensive sample characterization. In some cases, the density sensor 134 may include or be in thermal communication with the fourth heat exchanger 104d, which may help regulate the temperature of the density sensor 134 and/or the beverage sample 103, to help the density sensor 134 provide more accurate measurements.

In some instances, the first and second heat exchangers 104a, 104b may help regulate the temperature of an optical light path 116, which may include the one or more lenses 118 and the splitter 120, and/or the density sensor 134. In such instances, one or both of the third heat exchanger 104c and the fourth heat exchanger 104d may be omitted.

As previously stated, in some instances, the first and second heat exchangers 104a, 104b may be used to heat or cool one or more components of the density sensor 134 and/or the NIR spectrometer system 111 (e.g., the optical device 106). In certain instances, additional heat exchangers may be provided in the apparatus 100 to control the temperature of one or more components of the density sensor 134 and/or the NIR spectrometer system 111 (e.g., the optical device 106, the detector 132). For example, the apparatus 100 may include the fifth heat exchanger 104e in communication with the detector 132.

In certain instances, any of the aforementioned heat exchangers of the one or more heat exchangers 104 may be omitted from the apparatus 100 and/or a single heat exchanger of the one or more heat exchangers 104 may be in thermal communication with multiple components of the apparatus 100 (e.g., the third heat exchanger 104c may be in thermal communication with the optical device 106 and the density sensor 134). In certain instances, instead of each heat exchanger of the one or more heat exchangers 104 being provided with a heat sink, two or more of the heat exchangers may utilize the same heat sink. For example, the density sensor 134 and the optical device 106 may be in thermal communication with the same heat sink. As an additional example, a fan (not depicted) may be used to cool each of the heat sinks (and thus help regulate the temperature of the one or more heat exchangers 104).

In some instances, the third heat exchanger 104c may be used to regulate or control the temperature of the optical path, the one or more lenses 118, the splitter 120 (if provided), and/or the measurement cell 128. In such instances, the NIR spectrometer system 111 may produce more accurate spectra than if the temperature of the apparatus 100 and/or the optical light path 116 were not controlled or regulated.

In certain instances (e.g., if the fifth heat exchanger 104e is omitted), the third heat exchanger 104c may help regulate or control the temperature of the detector 132 of the NIR spectrometer system 111. In some cases, the third heat exchanger 104c and the fifth heat exchanger 104e may together help regulate or control the temperature of the detector 132 of the NIR spectrometer system 111. In various instances, the NIR spectrometer system 111 may produce more accurate spectra than if the temperature of the apparatus 100 and/or the NIR spectrometer system 111 were not controlled or regulated.

In some instances, the fourth heat exchanger 104d may be used to regulate or control the temperature of the beverage sample 103 provided to the density sensor 134.

The apparatus 100 may also be in fluid communication with a drain 138 such that the beverage sample 103 may be disposed of after measurements on the beverage sample are complete. Alternatively, if the apparatus 100 is provided in-line or on-line with a beverage manufacturing line, the drain 138 may be omitted and the beverage sample 103 may be returned to the beverage manufacturing line for further processing.

As noted above, the apparatus 100 may include the input conduit 108. The input conduit 108 may be in fluid communication with a source of a fluid such that, via actuation of the pump 109, the fluid may pass through the first and second heat exchangers 104a, 104b, the NIR spectrometer system 111 (i.e., the measurement cell 128 of the optical device 106), and the density sensor 134. The fluid may also pass through additional components (e.g., an additional heat exchanger) if such components are provided in the apparatus 100. Generally, the input conduit 108 is coupled to or in fluid communication with one or more fluid conduits (e.g., the fluid conduit 112) that provide a fluid flow path 127 for the beverage sample throughout the apparatus 100. The flow path of the fluid may terminate at an external sample container and/or the drain 138.

The pump 109 may be positioned on or otherwise in communication with the apparatus 100 and in operational communication with the input conduit 108 (as illustrated in FIGS. 1A and 1B) or, alternatively, an output conduit 110. The pump 109 may be configured or designed to help provide a pressure differential to move the beverage sample 103 out of the sample source 102 and facilitate the flow of the beverage sample 103 through the apparatus 100 until the beverage sample 103 is expelled through the drain 138.

Referring still to FIGS. 1A and 1B, the first and second heat exchangers 104a, 104b may each include one or more fluid conduits (e.g., the fluid conduit 112) or bores that provide the fluid flow path 127 through the first and second heat exchangers 104a, 104b. The fluid flow path 127 through each of the first and second heat exchangers 104a, 104b may be arranged in an S-shaped pattern, a spiral pattern, a coiled pattern, a helical pattern, or other similar patterns. The shape of the fluid flow path 127 through the first and second heat exchangers 104a, 104b may not only increase the amount of time that the beverage sample 103 is in contact with the first and second heat exchangers 104a, 104b (thereby helping to provide for more fine-tuned control of the temperature of the beverage sample 103), but also may allow the first and second heat exchangers 104a, 104b to be provided with a reduced size.

The various sensors provided with the apparatus 100 (e.g., the density sensor 134, the NIR spectrometer system 111) may be otherwise arranged in the apparatus 100. For example, the density sensor 134 may be provided before the NIR spectrometer system 111. In instances in which the density sensor 134 is provided before the NIR spectrometer system 111, re-pressurization of the fluid may be required. In such instances, a pump (not depicted) may be used to repressurize the fluid. In other such instances, a flow restrictor (not illustrated) configured or designed to maintain or raise the pressure of the fluid may be provided downstream of the density sensor 134.

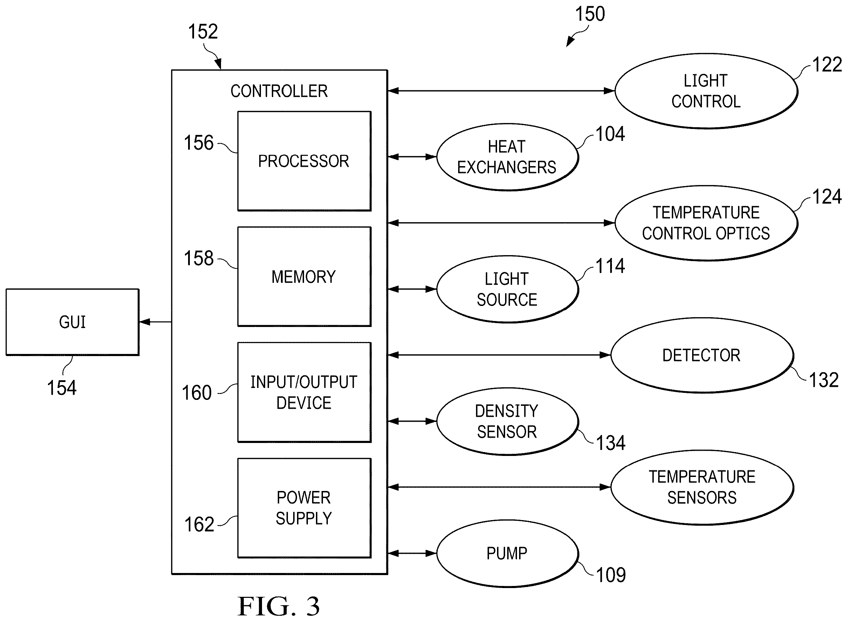

In some instances, the first and second heat exchangers 104a, 104b may also be in electrical communication with a heat control device 136 configured or designed to control the operation of the first and second heat exchangers 104a, 104b. The heat control device 136 may also be in electrical communication with a control system 150 (which may include a controller 152 and a GUI 154, as further explained with reference to FIG. 3) and may help the beverage sample within the apparatus 100 maintain a target temperature. In other instances, the heat control device 136 may be omitted and the operation of the first and second heat exchangers 104a, 104b may be determined by the control system 150. Data generated by one or more temperature sensors (not illustrated) positioned throughout the apparatus 100 may be used by the control system as input such that a controller 152 determines the operational parameters of the first and second heat exchangers 104a, 104b.

As can be seen in FIG. 1A, the beverage sample 103 may flow through the first and second heat exchangers 104a, 104b. Temperature affects the degree of hydrogen-bond clustering for water and ethanol with higher temperatures leading to smaller clusters on average and increased absorbance for free groups. As such, temperature has an indirect effect on the concentration analysis performed by the apparatus 100. However, the controller 152 may determine the identity of components of the beverage sample independent of this temperature effect using a procedure which uses a PLS regression model. In turn, the procedure which uses a PLS regression model may help the controller 152 of the apparatus 100 determine information related to the entire absorption spectrum generated by the apparatus 100, instead of only relying upon sections of the absorption spectrum that are not substantially impacted by temperature changes. In addition, the one or more heat exchangers 104 may help the apparatus 100 compensate for temperature effects.

An alternative arrangement of the optical device 106 is provided in FIG. 1B. In FIG. 1B, the optical device 106 still includes the light source 114, a light control mechanism 122, one or more lenses 118, an optics heat control device 124, and a measurement cell 128 and the NIR spectrometer system 111. However, unlike the optical device 106 illustrated in FIG. 1A, the splitter 120 has been omitted and the light control mechanism 122 has been repositioned. More specifically, the light control mechanism 122 is provided proximate or adjacent to the light source 114 such that the light from the light source 114 is provided directly to the light control mechanism 122 without the need for a component to change the direction of the light emanating from the light source 114.

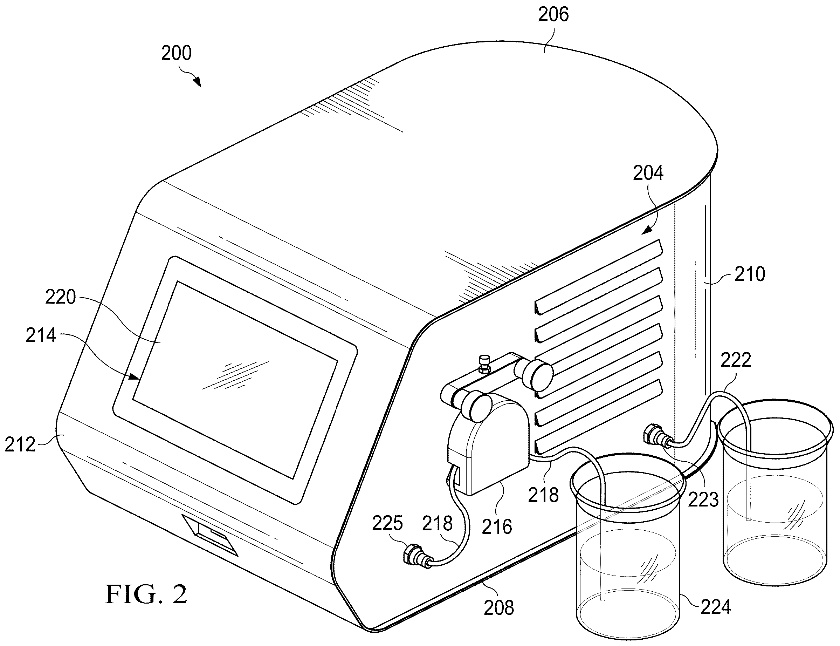

Referring now to FIG. 2, an apparatus for determining component concentrations of beverages 200 (hereinafter referred to as the "apparatus 200") may be provided in the form of a housing 204 including a top portion 206, a bottom portion 208, a sidewall 210, and a front wall 212, although the housing 204 may also include fewer components or additional components than those listed herein. The apparatus 200 may also include a pump 216 that is coupled to or in fluid communication with an inlet conduit 218. The apparatus 200 may also include an outlet conduit or output conduit 222 in fluid communication with an outlet 223 disposed in the housing 204. After analysis of a beverage sample is complete, the sample (or a portion thereof) may be provided to a drain (e.g., a drain 138, as shown in FIG. 1A) in fluid communication with the outlet 223 via the output conduit 222. In certain instances, the apparatus 200 may further include a user interface 214 and a display panel 220.

It is to be understood that the apparatus 200 is constructed using the same general principles as the apparatus 100 provided in FIGS. 1A and 1B. As such, elements of the apparatus 200 having similar names as components of the apparatus 100 may have a similar structure and function to the elements of the apparatus 100 as described above with reference to FIGS. 1A and 1B.

Referring still to FIG. 2, the top portion 206, the bottom portion 208, the sidewall 210, and the front wall 212 together may define an outer surface or outer shell of the housing 204. The housing 204 may be provided substantially in the form of a rectangular prism with rounded corners, although the housing 204 may be provided in other shapes (e.g., a cubic shape, an irregular three-dimensional shape, and an ovular shape). The top portion 206 may be located on the uppermost side of the housing 204 and may be provided in the form of a substantially flat covering. The bottom portion 208 may be located on the lowermost side of the housing 204 opposite the top portion 206 and may be provided in the form of a substantially flat base. The sidewall 210 may be positioned between the top portion 206 and the bottom portion 208 and may be provided in the form of a substantially U-shaped shell. The sidewall 210 may also house the pump 216 and the inlet conduit 218.

The pump 216 may be retained within a cylindrical housing operatively connected to the sidewall 210. In some instances, the pump 216 may be operatively connected to the inlet conduit 218. The inlet conduit 218 may be placed into fluid communication with a source of a beverage sample (e.g., a source 224). For example, the pump 216 and inlet conduit 218 may be configured or designed to extract or obtain beverage samples from the source 224 and provide the beverage samples to the apparatus 200 via an inlet 225.

In alternative instances, the pump 216 may be operatively connected to the output conduit 222. In such instances, the pump 216 may generate a pressure differential that causes the beverage samples to be drawn from the source 224, into the apparatus 200, and ultimately out of the apparatus 200 via the outlet 223. In yet other instances, the pump 216 may also be positioned elsewhere on, within, or external to the housing 204.

The front wall 212 may be located at the frontmost side of the housing 204 and may include the user interface 214. The user interface 214 (and thus the display panel 220) may be provided in the form of a flat, rectangular touchscreen, although the user interface 214 may also be provided in other shapes (e.g., square, triangle, oval, and teardrop shapes). The user interface 214 may be configured or designed to facilitate the operation of the apparatus 200 and to provide information about the apparatus 200. For example, the user interface 214 may allow a user to power the apparatus 200 on and/or off, adjust and select settings related to determining concentrations of or the identities of the components of a beverage sample, select an amount of a beverage sample to analyze, and/or to view or analyze results generated by the apparatus 200. In some instances, the display panel 220 may be an LED, LCD, or OLED display. For example, the display panel 220 may be provided in the form of a high-definition multimedia thin-film-transistor LCD ("HDMI TFT LCD") display.

Referring now to FIG. 3, the apparatus 100, 200 of FIGS. 1A, 1B, and 2 may include and be in communication with a control system 150, as shown in FIG. 3. For the sake of convenience, the description of the control system 150 generally refers to the components of the apparatus 100 of FIGS. 1A and 1B; however, it is to be understood that the description of the functionality of the control system 150 provided herein also applies to the apparatus 200 of FIG. 2.

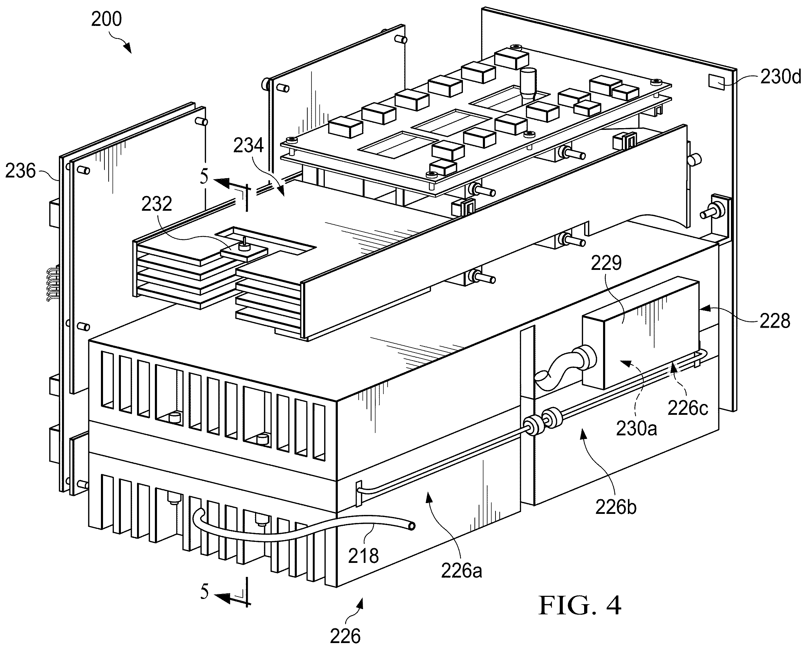

Referring again to FIG. 3, the control system 150 may include the controller 152 and the graphic user interface (GUI) 154. In various instances, the controller 152 may be provided "on board" in the apparatus 100, 200 and may be housed or disposed within the apparatus 100, 200. Alternatively, the controller 152 may be remote from the apparatus 100, 200. As shown in FIG. 3, the controller 152 may be electronically connected to and in electronic communication with the GUI 154. In some instances, the GUI 154 may be substantially the same as the user interface 214 of FIG. 2, or the GUI 154 may be implemented on the user interface 214. The controller 152 may also be electronically connected to and in electronic communication with one or more of the components of the apparatus 100, including one or more heat exchangers 104 (e.g., the first heat exchanger 104a and the second heat exchanger 104b of FIGS. 1A and 1B), the pump 109, the light source 114, the light control mechanism 122, the optics heat control device 124, the detector 132, the density sensor 134, the heat control device 136 (see FIGS. 1A and 1B), one or more temperature sensors (e.g., a first temperature sensor 230a of FIG. 4), and/or other components of the apparatus 100, 200. The controller 152 may also be electronically connected to the various components via a communications network. The communications network may be a wireless network such as a personal area network ("PAN") or a local area network ("LAN"), a cellular network, and/or the Internet.

Referring still to FIG. 3, the controller 152 may be Bluetooth enabled and have Internet of Things ("IoT") connectivity. The components of the apparatus 100 (e.g., the sensors, heat exchangers, light sources, and/or pump) may be IoT-enabled and/or communicatively connected smart components.

The controller 152 may send or receive electronic signals from one or more of the components of the apparatus 100 including the one or more heat exchangers, the light source 114, the density sensor 134, the pump 109, the light control mechanism 122, the optics heat control device 124, the detector 132, and/or the heat control device 136 (as shown in FIGS. 1A and 1B). The electronic signals received from one or more of the components may provide measurements and other data regarding one or more of absorption spectra, flow rate, pH level, alcohol by volume content, sugar content, carbohydrate content, the temperature of the sample, and/or other parameters. The measurements and other data may be sent to the controller 152 by the one or more components continuously, frequently, or periodically.

Referring still to FIG. 3, the control system 150 may use the measurements or other data received from one or more of the components of the apparatus 100 to send electronic signals to other components of the apparatus 100. In some instances, the controller 152 may send an electronic signal to one or more heat exchangers (e.g., the first and second heat exchangers 104a, 104b) to increase or decrease a temperature at which the one or more heat exchangers operate.

The controller 152 may include electronic components such as one or more processors 156, a memory 158 (e.g., random access memory ("RAM")), an input/output device 160, and a power supply 162 (e.g., battery or AC adapter plug). The controller 152 may be able to download, store, and/or execute software having computer-executable instructions. The software may include one or more modules. The one or more modules may include, for example, algorithms to monitor and/or store the measurements or other data received from one or more of the system components such as the sensors, heat exchangers, light source, temperature management devices, and/or pump. In some instances, the one or more modules may include, for example, algorithms to monitor and/or store real-time and historical usage data. The controller 152, via the one or more modules, may also perform calculations or other data analysis or modeling processes to determine various outcomes. For example, the controller 152 may include a procedure which uses a PLS regression model configured or designed to determine the identity of and/or the concentration of components of a beverage sample. In other instances, the controller 152 may also utilize other multivariate principal component regression models, including, but not limited to, a locally weighted regression ("LWR") model, a support vector machine regression ("SVM") model, and an artificial neural network ("ANN") model. Output determined by the controller 152 may include, for example, turning one or more of the system components of the apparatus 100 on or off at certain times or intervals, placing one or more of the system components in a standby mode, and/or providing information related to the "fingerprint" or identity of a beverage sample.

In some instances, the one or more modules may include a module configured or designed to analyze the raw data generated by the NIR spectrometer to produce absorption spectra. For example, the controller 152 may employ DD-SIMCA to determine, for example, the ABV of a test set of unknown samples. In some instances, the one or more modules may include an analysis module configured or designed to analyze obtained NIR absorption spectra using PLS regression models to determine the compounds comprising a beverage sample, the concentrations of the components, and ultimately the beverage sample's "fingerprint." In turn, the fingerprint may be used by the controller 152 to determine the identity of the sample using a reference fingerprint previously obtained from a known beverage sample. In addition, the output of the controller 152 may help determine when a quality of a beverage falls below a certain threshold and adjustments should be made with respect to the beverage production process to return the beverage to its target quality.

In some instances, the PLS regression method uses a linear relationship between the alcohol concentration and the spectral data. In such instances, the alcohol content of a beverage sample may be expressed using volume-by-volume concentration units (%v/v) and the light intensities may be expressed as absorbance values rather than the measured transmission values. Equations 1 through 5 demonstrate that absorbance A has such a linear relationship with the alcohol concentration $c_{alc}$ and that absorbance can be determined using the transmission spectrum of a beer ($T_B$) and water ($T_W$).

Equation 1 is a form of the Lambert-Beer law relating the light transmission T through a medium to the travel distance d and the attenuation coefficient $\mu$, which is a property of the medium. Equation 2 shows that in mixtures such as beer, $\mu$ can be written as a sum of the $\mu$-values of the separate components. For each species, $\mu$ is also expressed as the product of the concentration c and the molar attenuation coefficient $\varepsilon$ of the species. In this approximation, alcohol and water are considered the main components. However, the many different types of molecules in beer, including sugars, acids, oils, proteins, and more all contribute to a complex mixture. Applying Equations 1 and 2, the transmission for beer ($T_B$) and water ($T_W$) can be found, as reflected in Equations 3 and 4. Finally, as illustrated in Equation 5, the absorbance A of a beer sample is found to be proportional to the concentration of alcohol.

$$T = \frac{I}{I_0} = 10^{-\mu d} \qquad (1)$$

$$\mu_B \approx \mu_{alc} + \mu_{water} = \epsilon_{alc}\, c_{alc} + \epsilon_{water}\, c_{water} \qquad (2)$$

$$T_B = 10^{-\mu_{water}d} * 10^{-\mu_{alc}d} \qquad (3)$$

$$T_W = 10^{-\mu_{water}d} \qquad (4)$$

$$A = -\log_{10}\left(\frac{T_B}{T_W}\right) = -\log_{10}\left(10^{-\mu_{alc}d}\right) = \mu_{alc}d = \epsilon_{alc}c_{alc}d \qquad (5)$$

$$\longrightarrow A \propto c_{alc}$$

In some instances, the controller 152, utilizing the processor 156, may determine the absorbance value (A) in a manner consistent with the approach illustrated by Equations 1-5.

The detector 132 provided with the apparatus 100 may be configured or designed to measure the intensity of light with NIR wavelengths provided to the detector (e.g., the light transmission T). For example, the detector 132 may be configured or designed to measure the intensity of light with a wavelength of about 800 nm to about 2500 nm (or 800 nm to 2500 nm), or about 1000 nm to about 2200 nm (or 1000 nm to 2200 nm), or about 1250 nm to about 1040 nm (or 1250 nm to 1040 nm), or about 1550 nm to about 1950 nm (or 1550 nm to 1950 nm). Any information obtained by the detector 132 during sampling can be provided to the controller 152 for data processing and analysis such that absorption spectra can be generated and/or information related to the absorption spectra (e.g., component identity of the sample, component concentration of the sample, and the like) can be determined.

Still referring to FIG. 3, in some instances, machine learning (ML), artificial intelligence (AI), or similar processes may be implemented to iteratively train the control system 150 or the controller 152 and improve the performance of the apparatus based on one or more feedback parameters, characteristics, or similar information. For example, in some instances, ML/AI may be used to predict an optimal sample testing interval or pH/concentration threshold values. In some instances, ML/AI may be used to provide accurate sample testing and/or predict analyte value trends. Thus, the apparatus 100 may be optimized to efficiently test one or more beverage samples.

Referring now to FIG. 4, the internal components of the apparatus 200 of FIG. 2 are shown. The internal components may include a light source 232, a first and second heat exchangers 226a, 226b, a density sensor 228, an NIR spectrometer system 234, and a printed circuit board 236 ("PCB 236"), which may include the controller 152 of FIG. 3. In addition, the apparatus 200 may include various fluid conduits (not illustrated) configured or designed to place the first and second heat exchangers 226a, 226b, the density sensor 228, and NIR spectrometer system 234 in fluid communication with a beverage sample (not depicted), the inlet conduit 218, the outlet 223, and each other. As described with reference to FIG. 1A, the internal conduits may define a fluid flow path of the beverage sample through the apparatus 200.

Referring again to FIG. 4, the light source 232 may be provided in the form of a halogen lamp, a tungsten-halogen lamp, a krypton lamp, or any other suitable light source capable of producing light imparted with a wavelength of about 800 nm to about 2500 nm (or 800 nm to 2500 nm), although the light may also be imparted with a wavelength that is less than or greater than these values. For example, the light source 232 may produce light imparted with a wavelength of at least about 800 nm, or at least about 1000 nm, or at least about 1200 nm, or at least about 1400 nm, or at least about 1600 nm, or at least about 1800 nm, or at least about 2000 nm, or at least about 2200 nm, or at least about 2400 nm, or no more than about 2500 nm. As an additional example, the light source 232 may produce light imparted with a wavelength of at least 800 nm, or at least 1000 nm, or at least 1200 nm, or at least 1400 nm, or at least 1600 nm, or at least 1800 nm, or at least 2000 nm, or at least 2200 nm, or at least 2400 nm, or no more than 2500 nm.

NIR spectroscopy utilizes light transmission and absorption to measure various chemical and physical properties of a fluid sample, including the identity of and concentration of constituents in a sample material. The light source 232 of apparatus 200 may act as the source for the light transmission and absorption to aid in the determination of, for example, one or more of an alcohol concentration, a concentration of specific sugars, a concentration of aromatics, a concentration of bitterness-imparting compounds, and/or a concentration of carbohydrates in a beverage sample. In some instances, the light source 232 may act as the source for the light transmission and absorption to aid in the determination of a concentration of alcohol and a concentration of extract in the beverage sample.

The apparatus 200 may include one or more heat exchangers, such as the first and second heat exchangers 226a, 226b. The first and second heat exchangers 226a, 226b may each be in fluid communication with the beverage sample via the pump 216 (not illustrated) and the inlet conduit 218 (not illustrated). Furthermore, a conduit (not illustrated) may be positioned to place the first and second heat exchangers 226a, 226b in communication with each other, or the first and second heat exchangers 226a, 226b may be directly coupled together. Each of the first and second heat exchangers 226a, 226b may be configured or designed to heat or cool fluids (e.g., the beverage sample) as the fluid flows through the apparatus 200.

Generally, each of the one or more heat exchangers 226 provided in the apparatus 200 may be provided in the form of a temperature control device, a temperature sensor, and a heat sink. The one or more heat exchangers 226 may be provided as any temperature control device known in the art, including, but not limited to, a heating element, a strip heater, a ceramic heating element, PTC heating elements, Peltier elements, and the like. For example, the heating device of the first and second heat exchangers 226a, 226b may be provided as a Peltier element. Similarly, the temperature sensor may be provided as any temperature sensor known in the art, including, but not limited to, a resistance temperature sensor, an infrared temperature sensor, a thermistor, a thermocouple, and the like. In addition, the heatsink may be provided as any heat sink known in the art, including a passive heat sink and an active heat sink. For example, the heat sinks may be provided in the form of an extruded heat sink, a bonded heat sink, a skived heat sink, a stamped heat sink, a forged heat sink, a machined heat sink, and/or a fan. In certain instances, the heat sink may be composed of a metal with a high thermal conductivity value. For example, the heat sink may be composed of aluminum or copper.

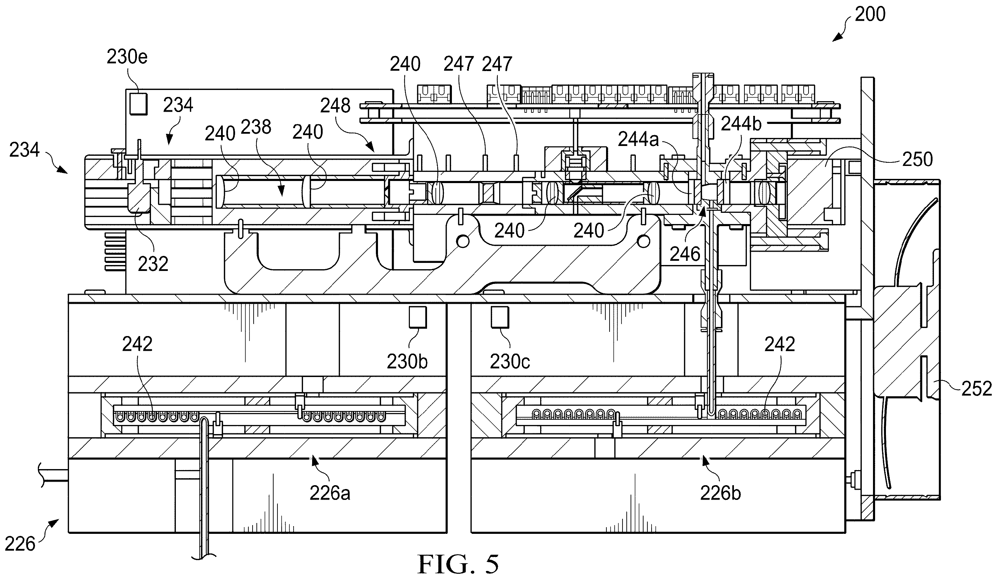

Referring now to FIG. 5, in certain instances, the first and second heat exchangers 226a, 226b may each be provided or in thermal communication with a temperature sensor. For example, the first heat exchanger 226a may be provided with a first temperature sensor 230a, and the second heat exchanger 226b may be provided with a second temperature sensor 230b. The first and second temperature sensors 230a, 230b may determine the temperature of the first and second heat exchangers 226a, 226b, respectively, and provide an input to a controller (such as controller 152 of FIG. 3) corresponding to the measured temperature of the first and second heat exchangers 226a, 226b.

Each of the first and second heat exchangers 226a, 226b may be provided in the form of a rectangular prism, although other forms and shapes for the first and second heat exchangers 226a, 226b are also contemplated. The first and second heat exchangers 226a, 226b may be positioned in a lower portion of the apparatus 200, although the first and second heat exchangers 226a, 226b may also be positioned elsewhere in the apparatus 200. The first and second heat exchangers 226a, 226b may be in fluid communication with each other and may be configured or designed to impart a beverage sample with a threshold or predetermined temperature.

The first and second heat exchangers 226a, 226b may transfer heat from one medium to another (e.g., air flowing through the first and second heat exchangers 226a, 226b and the beverage sample). For example, in some instances, the first and second heat exchangers 226a, 226b may transfer heat to and from the sample fluid provided to the apparatus 200. In addition, the first and second heat exchangers 226a, 226b can be used in both heating and cooling processes (e.g., to warm or cool the beverage sample provided to the apparatus 200 to a desired temperature). In some instances, the beverage sample may be heated and/or cooled until the beverage sample is imparted with a target temperature of about 10 °C to about 45 °C, or about 15 °C to about 35 °C, or about 17 °C to about 30 °C, or about 20 °C to about 25 °C, or about 20 °C, or about 25 °C. For example, the sample may be heated and/or cooled until the beverage sample is imparted with a target temperature of at least about 10 °C, or at least about 15 °C, or at least about 20 °C, or at least about 25 °C, or at least about 30 °C, or at least about 35 °C, or at least about 40 °C, or at least about 45 °C. As an additional example, the beverage sample may be heated and/or cooled until the beverage sample is imparted with a target temperature of 10 °C to 45 °C, or 15 °C to 35 °C, or 17 °C to 30 °C, or 20 °C to 25 °C, or 20 °C, or 25 °C. For example, the sample may be heated and/or cooled until the beverage sample is imparted with a target temperature of at least about 10 °C, or at least 15 °C, or at least 20 °C, or at least 25 °C, or at least 30 °C, or at least 35 °C, or at least 40 °C, or at least 45 °C. It is to be appreciated that the target temperature may be imparted with a value, or a range of values, falling between any minimum and maximum value recited herein.

The first and second heat exchangers 226a, 226b may be arranged in a serial configuration where the beverage sample is first provided to the first heat exchanger 226a before being provided to the second heat exchanger 226b. In some such instances, the second heat exchanger 226b may be positioned downstream of the first heat exchanger 226a, although the first heat exchanger 226a may also be positioned downstream of the second heat exchanger 226b. In addition, the first and second heat exchangers 226a, 226b may be configured or designed to heat or cool the beverage sample within a defined range or a defined tolerance relative to a target temperature. For example, the first heat exchanger 226a may be configured or designed to heat or cool a sample within about 1 °C to about 10 °C of a target temperature and the second heat exchanger 226b may be configured or designed to heat or cool a sample within about 0 °C to about 1 °C of a target temperature. As an additional example, the first heat exchanger 226a may be configured or designed to heat or cool a sample within about 0.5 °C to about 2 °C of the target temperature and the second heat exchanger 226b may be configured or designed to heat or cool the sample within about 0 °C to about 0.75 °C of the target temperature. As yet another example, the first heat exchanger 226a may be configured or designed to heat or cool a sample to within about 0 °C to about 1.5 °C of a target temperature and the second heat exchanger 226b may be configured or designed to heat or cool a sample within about 0 °C to about 0.5 °C of a target temperature. In other instances, the first heat exchanger 226a may be configured or designed to heat or cool a sample within 1 °C to 10 °C of a target temperature and the second heat exchanger 226b may be configured or designed to heat or cool a sample within 0 °C to 1 °C of a target temperature. As an additional example, the first heat exchanger 226a may be configured or designed to heat or cool a sample within 0.5 °C to 2 °C of the target temperature and the second heat exchanger 226b may be configured or designed to heat or cool the sample within 0 °C to 0.75 °C of the target temperature. As yet another example, the first heat exchanger 226a may be configured or designed to heat or cool a sample to within 0 °C to 1.5 °C of a target temperature and the second heat exchanger 226b may be configured or designed to heat or cool a sample within 0 °C to 0.5 °C of a target temperature.

It is to be appreciated that the first heat exchanger 226a may be configured or designed to heat or cool a sample within any range of temperatures having a minimum value and maximum value described herein with respect to the first heat exchanger 226a. It is also to be appreciated that the second heat exchanger 226b may be configured or designed to heat or cool a sample within any range of temperatures having a minimum value and maximum value described herein with respect to the second heat exchanger 226b.

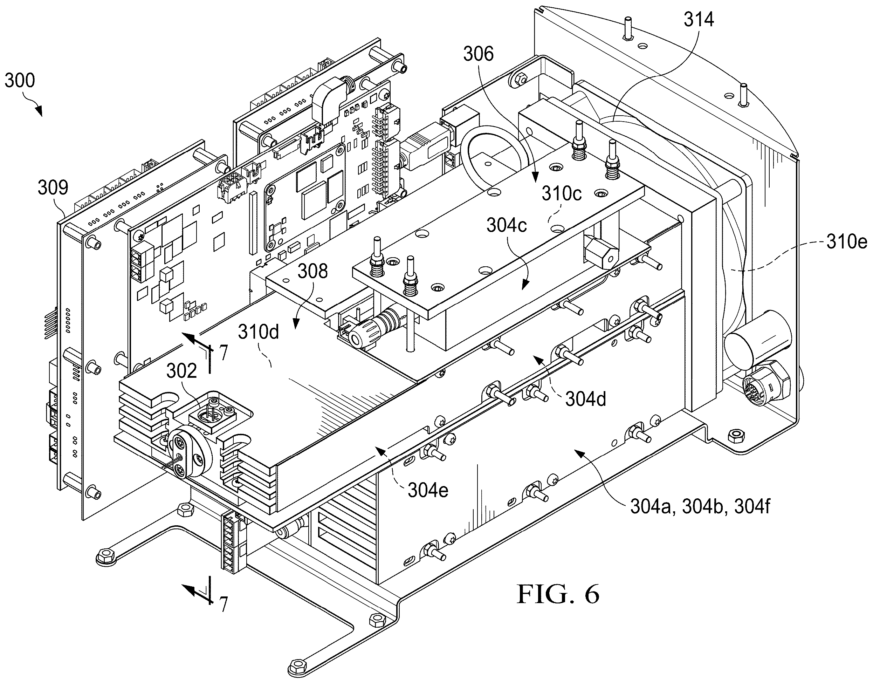

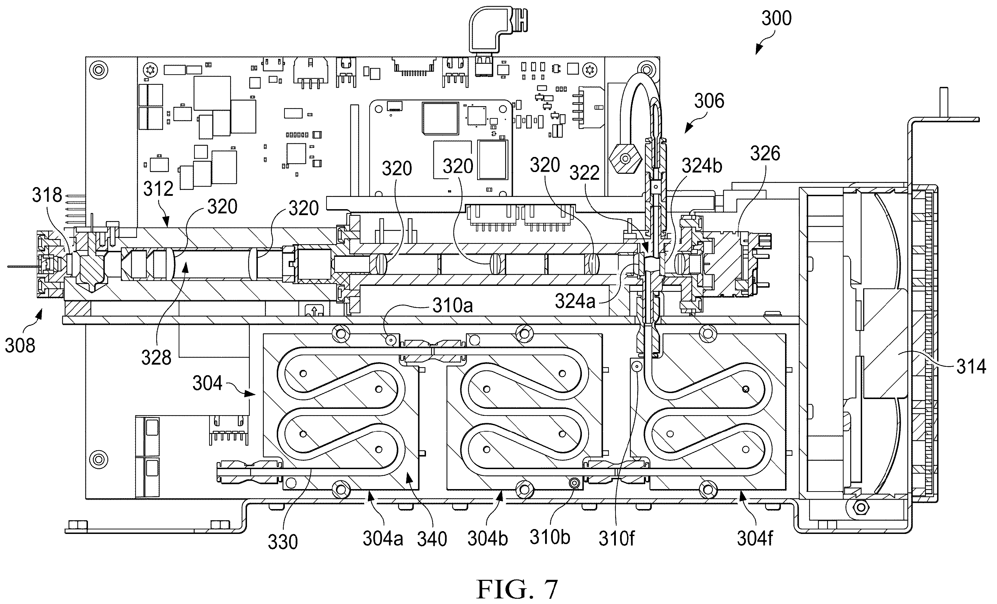

In some instances, the apparatus 100 may be provided with an additional heat exchanger (see, e.g., a sixth heat exchanger 304f of an apparatus 300 of FIGS. 6 and 7) that is in fluid communication with the first and second heat exchangers 226a, 226b. In such instances, the additional heat exchanger may be serially coupled to the first and second heat exchangers 226a, 226b. For example, the beverage sample may be provided first to the first heat exchanger 226a before being provided to the second heat exchanger 226b before ultimately being provided to the additional heat exchanger. Stated another way, the additional heat exchanger may be arranged downstream of the first and second heat exchangers 226a, 226b, although the aforementioned heat exchangers may also be arranged in other configurations. In such instances, the additional heat exchanger may be configured or designed to heat or cool a sample to within about 0 °C to about 0.1 °C of a target temperature. For example, the additional heat exchanger may heat or cool the sample to within about 0.1 °C, or within about 0.05 °C, or within about 0.04 °C, or within about 0.03 °C, or within about 0.02 °C, or within about 0.01 °C, or within about 0 °C of the target temperature. In other instances, the additional heat exchanger may be configured or designed to heat or cool a sample to within 0 °C to 0.1 °C of a target temperature. For example, the additional heat exchanger may heat or cool the sample to within 0.1 °C, or within 0.05 °C, or within 0.04 °C, or within 0.03 °C, or within 0.02 °C, or within 0.01 °C, or within 0 °C of the target temperature.

It is to be appreciated that the additional heat exchanger may be configured or designed to heat or cool a sample within any range of temperatures having a minimum value and maximum value described herein with respect to the additional heat exchanger.

In certain instances, the additional heat exchanger may be provided with or in communication with an additional temperature sensor (not illustrated). In such instances, the additional temperature sensor may be configured or designed to monitor the temperature of the additional heat exchanger and provide an input to the controller 152 associated with the temperature of the additional heat exchanger. In some instances, the apparatus 200 may be provided with fewer heat exchangers than described herein. In such instances, the beverage sample may still be heated or cooled to within a defined tolerance of a target temperature in a manner consistent with the teachings herein.

In some instances, the first and second heat exchangers 226a, 226b can provide substantially stable temperature control to help the apparatus 200 provide accurate measurements (e.g., absorption spectra, density values). For instance, when multiple samples are analyzed by the apparatus 200 at different times, a temperature variation of the beverage samples provided to the apparatus 200 at different times may be within about 0 °C to about 1 °C, or about 0 °C to about 0.1 °C, or about 0.001 °C to about 0.05 °C, or about 0.001 °C to about 0.01 °C. In certain instances, the temperature variation of the beverage samples provided to the apparatus 200 at different times may be within 0 °C to 1 °C, or 0 °C to 0.1 °C, or 0.001 °C to 0.05 °C, or 0.001 °C to 0.01 °C. It is to be appreciated that the additional heat exchanger may also be used in conjunction with the first and second heat exchangers 226a, 226b to provide the stable temperature control.

Referring back to FIG.4, the density sensor 228 may be located adjacent to the first and second heat exchangers 226a, 226b although the density sensor 228 may also be provided elsewhere within the apparatus 200. A housing 229 of the density sensor 228 may be provided in the form of a rectangular prism, although the housing 229 of the density sensor 228 may also be provided in other shapes and forms. Relative to the fluid flow path of the beverage sample through the apparatus 200, the density sensor 228 may be positioned downstream of the first and second heat exchangers 226a, 226b. In addition, the density sensor 228 may be positioned either upstream or downstream of the NIR spectrometer system 234. Preferably, the density sensor 228 is positioned downstream of the NIR spectrometer system 234 to help prevent the depressurization and degassing of the fluid before the fluid is provided to the NIR spectrometer system 234.

The density sensor 228 may be provided in the form of an oscillation-based density sensor, a vibrating element-based density sensor, or another suitable type of density sensor that can measure the density of a fluid. For example, the density sensor 228 may be provided in the form of an oscillation-based density sensor that utilizes the oscillation frequency principle to determine density. For example, a measuring cell (not illustrated) of the density sensor 228 may contain a vibrating element, such as a tuning fork or quartz crystal, which vibrates at a frequency that is proportional to the density of the fluid. The transducers of the density sensor 228 may then measure the oscillation's resonance frequency, which is impacted by the bulk and density of the sample within the measuring cell. The measured resonant frequency may be compared to a known reference frequency derived from a reference sample or calibration standards to determine the density of the sample. In comparison, a vibrating element-based density meter uses the damping effect that occurs on a vibrating element when the vibrating element is immersed in a fluid. For example, a vibrating element, such as a quartz crystal or a tuning fork, is set in motion at a given frequency by the density sensor 228. Sensors or transducers within the density sensor 228 may then measure the shift in frequency of the vibrating element induced by the damping effect of the sample fluid. The density sensor 228 may be calibrated using known density reference standards to create a relationship between frequency shift and density. Using the calibration data, the density sensor 228 subsequently transforms the frequency shift into a matching density value.

As an additional example, the density sensor 228 may also be provided in the form of a measurement device that determines the resonance frequency of a conduit (e.g., a U-shaped tube) within the apparatus when determining the density of the fluid sample. In some instances, the density sensor 228 may also measure other parameters of the beverage sample, such as the specific gravity of the sample. In various instances, the density sensor 228 may be imparted with high accuracy, compact packaging, and provide a fast response time when determining density values of the beverage sample.

It is to be appreciated that the density sensor 228 may be provided in other forms than those described herein.

In certain instances, a third heat exchanger 226c may be in thermal communication with the density sensor 228. In such instances, the third heat exchanger 226c may help regulate or control the temperature of the density sensor 228 (and thus the fluid flowing through the density sensor 228). Thus, the density sensor 228 may produce more accurate density readings than if the temperature of the beverage sample was not controlled or regulated. In certain instances, the third heat exchanger 226c may impart the density sensor 228 (and/or the fluid within the density sensor 228) with a temperature of about 10 °C to about 30 °C (or 10 °C to 30 °C), or about 15 °C to about 25 °C (or 15 °C to 25 °C), or about 20 °C to about 25 °C (or 20 °C to 25 °C). For example, the third heat exchanger 226c may impart the density sensor 228 (and/or the fluid within the density sensor 228) with a temperature of at least about 10 °C, or at least about 15 °C, or at least about 20 °C, or at least about 25 °C, or at least about 30 °C. As an additional example, the third heat exchanger 226c may impart the density sensor 228 (and/or the fluid within the density sensor 228) with a temperature of at least 10 °C, or at least 15 °C, or at least 20 °C, or at least 25 °C, or at least 30 °C. In other instances, the third heat exchanger 226c may impart the density sensor 228 with a temperature that is higher or lower than the values recited herein.

The third heat exchanger 226c may be configured or designed to impart the density sensor 228 (and/or the fluid within the density sensor 228) with a temperature that is within a defined threshold or tolerance. In some instances, the tolerance of the third heat exchanger 226c may be within a range of about -1 °C to about 1 °C (or -1 °C to 1 °C). For example, the tolerance of the third heat exchanger 226c may be no more than about ±1 °C, or no more than about ±0.5 °C, or no more than about ±0.4 °C, or no more than about ±0.3 °C, or no more than about ±0.2 °C. In other instances, the tolerance of the third heat exchanger 226c may be somewhat higher or lower than the values recited herein.

In some instances, a third temperature sensor 230c may be provided with or in thermal communication with the density sensor 228 and/or the third heat exchanger 226c. In such instances, the third temperature sensor 230c may determine the temperature of the density sensor 228 and/or the third heat exchanger 226c and provide an input to the controller 152 corresponding to the temperature of the density sensor 228 and/or the third heat exchanger 226c. In some cases, each of the density sensor 228 and the third heat exchanger 226c may be provided with a temperature sensor. In such instances, the third temperature sensor 230c may be associated with the density sensor 228 and a fourth temperature sensor (not illustrated) may be associated with the third heat exchanger 226c.

Referring again to FIG. 4, the electronic components of the apparatus 200 may be in electrical communication with the PCB 236. The PCB 236 may include at least one embedded microcontroller (e.g., the controller 152 of FIG. 3). The microcontroller may include a processor and memory, which may be used to store, for example, pre-programmed functions associated with facilitating operation of the heat exchangers 226a-226e, the density sensor 228, the NIR spectrometer system 234, and/or modeling software (e.g., a PLS regression model).

Now referring again to FIG. 5, various internal components of the apparatus 200 are shown. As previously mentioned, the light source 232 may be positioned and located anywhere in the apparatus 200. The light source 232 may be a halogen lamp or any other suitable light source. The apparatus 200 may further include a measurement cell 246 and a detector 250. In some instances, the detector 250 may be located adjacent to the measurement cell 246 and the measurement cell 246 may be arranged such that light generated by the light source 232 may pass through a first window 244a and a second window 244b before being provided to the detector 250.

Referring again to FIG. 5, in some instances, the light source 232 and the detector 250 may be positioned at opposite ends of the NIR spectrometer system 234 within the apparatus 200. In such instances, an optical light path 238 of the light source 232 may be substantially linear (see also the optical light path 116 of FIG. 1A). When the optical light path 238 is not substantially linear, reflectors or mirrors (not illustrated) may be positioned such that the light generated by the light source 232 may change direction and be provided to the location where the detector 250 is positioned.

In some instances, an optical device 248 and a splitter 247 may be positioned adjacent to one or more lenses 240 and may be positioned such that the light generated by the light source 232 may be split into two or more beams by the splitter 247. In other instances, such as the apparatus 100 provided in FIG. 1B, the splitter 247 may be omitted.

The first and second heat exchangers 226a, 226b may include a bore or fluid conduit 242 that provides a fluid flow path for the beverage sample through the first and second heat exchangers 226a, 226b. Providing the fluid conduit 242 as a spiral, helical, or coil-shaped conduit in the first and second heat exchangers 226a, 226b may help the first and second heat exchangers 226a, 226b to be constructed more compactly and facilitate heating and cooling of both sides of the sample in the first and second heat exchangers 226a, 226b. However, the fluid conduit 242 may also be provided in other shapes and forms besides those listed herein.

Referring still to FIG. 5, in some instances, regulating the temperature surrounding the apparatus 200 may help control the temperature of the beverage sample. In such instances, a fan 252 may be provided in the apparatus 200 to cool the heated parts of the apparatus 200, for example, the heat exchangers 226a-226e, the light source 232, and/or the NIR spectrometer system 234.

In certain instances, a fourth temperature sensor 230d may be provided in the apparatus 200. The fourth temperature sensor 230d may be configured or designed to monitor the overall temperature of the apparatus 200. Measurements obtained from the fourth temperature sensor 230d may be utilized by a control system, such as the control system 150 of FIG. 3, to determine when the fan 252 should be activated to cool components of the apparatus 200 and/or when the fan 252 should be deactivated. In certain cases, the apparatus 200 may include additional temperature sensors or fewer temperature sensors than those described herein.

In some instances, the apparatus 200 may combine the NIR spectrometer system 234 with an additional sensor (not illustrated) configured or designed to determine the concentration of multiple components in the beverage samples, including $CO_2$, aromatics, and components that provide bitterness. For example, the apparatus 100 may optionally include a mid-infrared attenuated total reflectance (MIR-ATR) sensor that is configured or designed to determine the concentration of multiple components of the beverage sample, including $CO_2$, aromatics, and components that provide bitterness. In certain instances, the additional sensor may be configured or designed to measure the $CO_2$ concentration of the beverage sample. For example, the apparatus 200 may include a pH probe, a thermal conductivity sensor and/or an infrared sensor (e.g., a MIR-ATR sensor) configured or designed to determine the $CO_2$ concentration in the beverage sample. Including the additional sensor configured or designed to determine the $CO_2$ concentration in the beverage sample may help compensate for any impact the dissolved $CO_2$ has on the absorption spectra and/or the density measurements generated by the apparatus 200.

In instances in which the additional sensor is provided, the additional sensor may be positioned and located between the measurement cell 246 and the density sensor 228. In other instances, the additional sensor may be provided elsewhere in the apparatus 200 (e.g., in the path of the light generated by the light source 232 or in the fluid flow path within or outside of the apparatus 200). In instances in which the additional sensor is not in fluid communication with the one or more heat exchangers of the apparatus 200, the additional sensor may be provided with a heat exchanger.

Turning to FIGS. 6 and 7, an alternative configuration for the internal components of the apparatus 200 (the alternative configuration hereinafter referred to as the "apparatus 300") is provided. Components of the apparatus 300 imparted with similar names and/or numbering as compared to the components of the apparatus 100, 200 of FIGS. 1A, 1B, 2, 4 and 5 may have a substantially similar structure and function as the similarly named and numbered components of the apparatus 200. Like in the instance of the apparatus 200, the apparatus 300 may be provided with external components substantially similar to the external components of apparatus 200 depicted in FIG. 2. In addition, as illustrated in FIGS. 6 and 7, the apparatus 300 may also include a light source 302, one or more heat exchangers 304 (e.g., a first heat exchanger 304a, a second heat exchanger 304b, a third heat exchanger 304c, a fourth heat exchanger 304d, a fifth heat exchanger 304e, and a sixth heat exchanger 304f), a density sensor 306, a NIR spectrometer system 308, a PCB 309, one or more temperature sensors (e.g., a first temperature sensor 310a, a second temperature sensor 310b, a third temperature sensor 310c, a fourth temperature sensor 310d, a fifth temperature sensor 310e, a sixth temperature sensor 310f, etc.) an optical device 312, a light control mechanism 318, lenses 320, a measurement cell 322, measurement windows 324a and 324b, a fan 314, a detector 326, an optical light path 328, and a fluid conduit 330. However, unlike the apparatus 200 illustrated in FIGS. 2, 4, and 5, the apparatus 300 includes a sixth heat exchanger 304f and an associated temperature sensor (e.g., the sixth temperature sensor 310f) that are configured or designed to provide further temperature control of the beverage sample before the beverage sample is provided to the NIR spectrometer system 308. In addition, in comparison to the apparatus 100, 200, various components of the apparatus 300 may be positioned in alternative locations. For example, the fluid conduit 330 within the heat exchangers 304a, 304b, 304f may be arranged in a serpentine pattern to provide prolonged contact with the heat exchangers 304a, 304b, and 304f. As an additional example, the density sensor 306 may be provided near a top portion of the apparatus 300, instead of being provided on the side portion, as in the apparatus 200. Furthermore, in comparison to the apparatus 200, a splitter may be omitted in the optical light path 328. In some instances, the apparatus 300 may include additional components or fewer components than those described herein.

The heat exchangers 304a, 304b, 304f may be placed in fluid communication with one another. In some instances, the heat exchangers 304a, 304b, 304f may be arranged in a serial configuration wherein the beverage sample is first provided to the first heat exchanger 304a and then to the second heat exchanger 304b before being provided to the sixth heat exchanger 304f. In addition, the heat exchangers 304a, 304b, 304f may be configured or designed to heat or cool the beverage sample within a defined range or a defined tolerance relative to a target temperature. For example, the first heat exchanger 304a may be configured or designed to heat or cool a sample within about 1 °C to about 10 °C of a target temperature, the second heat exchanger 304b may be configured or designed to heat or cool a sample within about 0 °C to about 1 °C of a target temperature, and the sixth heat exchanger 304f may be configured or designed to heat or cool a sample within about 0 °C to about 0.1 °C of a target temperature. As an additional example, the first heat exchanger 304a may be configured or designed to heat or cool a sample within about 0.5 °C to about 2 °C of the target temperature, the second heat exchanger 304b may be configured or designed to heat or cool the sample within about 0 °C to about 0.75 °C of the target temperature, and the sixth heat exchanger 304f may be configured or designed to heat or cool a sample within about 0 °C to about 0.01 °C of the target temperature. As yet another example, the first heat exchanger 304a may be configured or designed to heat or cool a sample to within about 0 °C to about 1.5 °C of a target temperature, the second heat exchanger 304b may be configured or designed to heat or cool a sample within about 0 °C to about 0.5 °C of a target temperature, and the sixth heat exchanger 304f may be configured or designed to heat or cool a sample within about 0 °C to about 0.001 °C of the target temperature.

In some instances, the first heat exchanger 304a may be configured or designed to heat or cool a sample within 1 °C to 10 °C of a target temperature, the second heat exchanger 304b may be configured or designed to heat or cool a sample within 0 °C to 1 °C of a target temperature, and the sixth heat exchanger 304f may be configured or designed to heat or cool a sample within 0 °C to 0.1 °C of a target temperature. In other instances, the first heat exchanger 304a may be configured or designed to heat or cool a sample within 0.5 °C to 2 °C of the target temperature, the second heat exchanger 304b may be configured or designed to heat or cool the sample within 0 °C to 0.75 °C of the target temperature, and the sixth heat exchanger 304f may be configured or designed to heat or cool a sample within 0 °C to 0.01 °C of the target temperature. In yet other instances, the first heat exchanger 304a may be configured or designed to heat or cool a sample to within 0 °C to 1.5 °C of a target temperature, the second heat exchanger 304b may be configured or designed to heat or cool a sample within 0 °C to 0.5 °C of a target temperature, and the sixth heat exchanger 304f may be configured or designed to heat or cool a sample within 0 °C to 0.001 °C of the target temperature.

In some instances, the apparatus 300 may be provided with a first heat exchanger 304a that is configured or designed to heat or cool a beverage sample to within about 3 °C, or within about 2.5 °C, or within about 2 °C, or within about 1.9 °C, or within about 1.8 °C, or within about 1.7 °C, or within about 1.6 °C, or within about 1.5 °C, or within about 1.4 °C, or within about 1.3 °C, or within about 1.2 °C, or within about 1.1 °C, or within about 1 °C of a target temperature. In other instances, the apparatus 300 may be provided with a first heat exchanger 304a that is configured or designed to heat or cool a beverage sample to within 3 °C, or within 2.5 °C, or within 2 °C, or within 1.9 °C, or within 1.8 °C, or within 1.7 °C, or within 1.6 °C, or within 1.5 °C, or within 1.4 °C, or within 1.3 °C, or within 1.2 °C, or within 1.1 °C, or within 1 °C of a target temperature.

In some instances, the apparatus 300 may be provided with a second heat exchanger 304b that is configured or designed to heat or cool a beverage sample to within about 1.5 °C, or within about 1.4 °C, or within about 1.3 °C, or within about 1.2 °C, or within about 1.1 °C, or within about 1 °C, or within about 0.9 °C, or within about 0.8 °C, or within about 0.7 °C, or within about 0.6 °C, or within about 0.5 °C, or within about 0.4 °C, or within about 0.3 °C, or within about 0.2 °C, or within about 0.1 °C of a target temperature. In other instances, the apparatus 300 may be provided with a second heat exchanger 304b that is configured or designed to heat or cool a beverage sample to within 1.5 °C, or within 1.4 °C, or within 1.3 °C, or within 1.2 °C, or within 1.1 °C, or within 1 °C, or within 0.9 °C, or within 0.8 °C, or within 0.7 °C, or within 0.6 °C, or within 0.5 °C, or within 0.4 °C, or within 0.3 °C, or within 0.2 °C, or within 0.1 °C of a target temperature.

In some instances, the apparatus 300 may be provided with a sixth heat exchanger 304f that is configured or designed to heat or cool a beverage sample to within about 0.5 °C, or within about 0.4 °C, or within about 0.3 °C, or within about 0.2 °C, or within about 0.1 °C, or within about 0.05 °C, or within about 0.04 °C, or within about 0.03 °C, or within about 0.02 °C, or within about 0.01 °C, or within about 0 °C of a target temperature. In other instances, the apparatus 300 may be provided with a sixth heat exchanger 304f that is configured or designed to heat or cool a beverage sample to within 0.5 °C, or within 0.4 °C, or within 0.3 °C, or within 0.2 °C, or within 0.1 °C, or within 0.05 °C, or within 0.04 °C, or within 0.03 °C, or within 0.02 °C, or within 0.01 °C, or within 0 °C of a target temperature.

Now referring to FIG. 8, the representative beverage manufacturing system 800 is provided. In some instances, the apparatus 100, 200, 300 may be positioned in a process line that is utilized to manufacture alcoholic beverages such as beer. For example, the apparatus 100, 200, 300 may be positioned in-line or on-line within the beverage manufacturing system 800. In such instances, the apparatus 100, 200, 300 may be adapted such that the apparatus 100, 200, 300 may continuously or periodically sample the alcoholic beverage in the production line. Alternatively, samples may be removed from defined locations in the beverage manufacturing system 800 and provided to the apparatus 100, 200, 300 if the apparatus 100, 200, 300 is not positioned in-line or on-line within the beverage manufacturing system 800. In some instances, the beverage manufacturing system 800 may also be adapted to produce non-alcoholic beverages.

The beverage manufacturing system 800 may be provided with raw materials, such as raw materials 801, that are used to create the alcoholic beverage. In the illustrated instance, the raw materials 801 are provided in the form of malt, although other raw materials (e.g., materials used to create wine) may be provided to the beverage manufacturing system 800. In the illustrated instance, the beverage manufacturing system 800 is provided in the form of a malt silo 802, a milling apparatus 804, a mash tun 806, a lauter tun 808, a boiling apparatus 810, a whirlpool apparatus 812, a cooling apparatus 814, an aeration and yeast dosing apparatus 816, a fermentation tank 818, a storage tank 820, a centrifuge 822, a filtration apparatus 824, a carbonation and blending apparatus 826, a bright beer tank 828, a pasteurization apparatus 830, and a packaging system 832, each of the aforementioned components placed in fluid communication with one another by fluid conduits 834. As would be appreciated by one having skill in the art, the components of the beverage manufacturing system 800 may be alternatively arranged, additional components may be added, or some components may be omitted in alternative versions of the beverage manufacturing system 800. In addition, the beverage manufacturing system 800 may be configured or designed to produce other alcoholic beverages, such as wine.

In certain instances, the apparatus 100, 200, 300 may be positioned in-line or on-line within the beverage manufacturing system 800 at sample locations 840, although in some instances the apparatus 100, 200, 300 may be positioned in alternative locations in the beverage manufacturing system 800. Regardless of where the apparatus 100, 200, 300 is positioned and located, the apparatus 100, 200, 300 may sample a portion of the beverage to determine the concentration of selected components of the beverage and/or to determine if the beverage being produced is within defined tolerances.

Advantageously, in instances in which the apparatus 100, 200, 300 is provided in-line in the beverage manufacturing system 800, the apparatus 100, 200, 300 may continuously or periodically sample the beverage being produced by the beverage manufacturing system 800. In turn, this may allow beverage manufacturers to continuously or periodically monitor the quality and the composition of their alcoholic beverages during the production process, which in turn may allow the manufacturers to quickly identify when problems arise in the brewing process. In addition, in instances in which the beverage is being sampled continuously, the need for the apparatus 100, 200, 300 to be flushed between measurements is reduced or eliminated, which in turn conserves flush fluid and reduces the operational cost of the apparatus 100, 200, 300. Furthermore, using the apparatus 100, 200, 300 in-line eliminates the need for a worker to obtain a sample of the alcoholic beverage and provide the alcoholic beverage to the apparatus 100, 200, 300, reducing labor costs for the beverage manufacturer.

During the production process, the temperature of the beverage being produced may vary. Such fluctuations in temperature could be caused by, for example, changes in the ambient or environmental temperature of the brewery plant or changes in the brewery process. Thus, another advantage of providing the apparatus 100, 200, 300 in-line in the beverage manufacturing system 800 is that the apparatus 100, 200, 300 may compensate for such temperature variations via one or more methods described with reference to the apparatus 100, 200, 300 (e.g., by adjusting the temperature of the sampled beverage via heat exchangers before providing the sampled beverage to the NIR spectrometer system).

Referring still to FIG. 8, by focusing on the temperature shift of the beverage sample (e.g., by monitoring the temperature of the sample via a temperature sensor and adjusting the analysis of the absorption spectra by a degree corresponding with the measured temperature), the effects of the temperature on the data obtained by the apparatus 100, 200, 300 may be determined. As previously mentioned, the temperature of not only the sample but also of the apparatus 100, 200, 300 itself influences the absorption spectra generated from the beverage samples. As such, a computation module for temperature may be utilized (e.g., via the controller 152 of FIG. 3) in the analysis of the alcohol sample. The computation module for temperature may be carried out by the processor 156 of the controller 152 (see FIG. 3). This allows the beverage sample to be tested without temperature control, providing for a more efficient analysis of the beverage samples.

Advantageously, utilizing a temperature-calibration module (e.g., via the controller 152) allows for the entire NIR absorption spectra of the beverage samples to be analyzed, instead of only analyzing portions of the spectra that are not impacted by temperature. Thus, the apparatus 100, 200, 300 is capable of analyzing areas of the NIR spectrum where the temperature effects are normally observed. In turn, this may allow for the apparatus 100, 200, 300 to determine the identity of the components of the beverage sample and/or the concentration of the components more accurately and precisely.

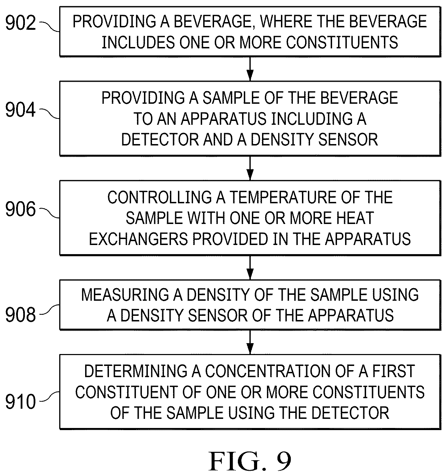

FIG. 9 is a flowchart of a method 900 for determining concentrations of one or more constituents of a beverage. The method 900 may be used with any of the apparatus 100, 200, 300, and variations thereof, disclosed herein.

The method 900 includes a step 902 of providing a beverage, where the beverage includes one or more constituents. The method 900 also includes a step 904 of providing a sample of the beverage to an apparatus including a detector and a density sensor. In some cases, the detector is part of an NIR spectrometer or an NIR spectrometer system.

The method 900 further includes a step 906 of controlling a temperature of the beverage with one or more heat exchangers associated with the apparatus. In some cases, the one or more heat exchangers include a first heat exchanger and a second heat exchanger. In some such cases, the one or more heat exchangers include a third heat exchanger. In various instances, the first and second heat exchanger (and, if provided, the third heat exchanger) may be arranged is series and positioned upstream of the detector to control the temperature of the beverage before the beverage is provided to the detector.

The method 900 also includes a step 908 of measuring a density of the beverage using a density sensor of the apparatus. In alternative instances, the step 908 may include determining specific gravity of the beverage.

The method 900 also includes a step 910 of determining a concentration of a first constituent of one or more constituents of the beverage using the detector. In other cases, the step 910 may include analyzing both the density and NIR spectra generated by the apparatus to determine a concentration of the first constituent. In some cases, a controller determines the concentration of the first constituent at least partially based on an absorption spectrum determined by the measurement device. In certain instances, the first constituent is selected from the group consisting of alcohol, sugar, extract, carbon dioxide, a total carbohydrate content, aromatics, proteins, and bitterness-imparting compounds. In some such instances, the first constituent is alcohol or extract.

In certain instances, the method 900 further includes determining an identity of the beverage utilizing an absorption spectrum created from data obtained from the measurement device.

In some instances, the beverage sample may be an alcoholic beverage, such as beer, spirits, wine, and the like. In other instances, the beverage sample may be a non-alcoholic beverage that is completely free or substantially free of alcohol (e.g., non-alcoholic beer, non-alcoholic spirits, non-alcoholic wine). In certain instances, the method 900 may include a step of determining an alcohol content of a non-alcoholic beverage to determine whether the alcohol content is below a determined threshold (e.g., 0.5% abv).

In some instances of the method 900, a controller determines the concentration of the first constituent at least partially based on an absorption spectrum generated by the NIR spectrometer.

In other instances of the method 900, the apparatus further includes a controller having a memory configured or designed to receive data from the density sensor and the NIR spectrometer, and the controller includes a processor configured or designed to execute a procedure which uses a PLS regression model.

In some instances, the method 900 also includes a step of determining a concentration of a second constituent of the plurality of constituents. In other instances, the method 900 further includes a step of determining an identity of a beverage sample utilizing an absorption spectrum created from data obtained from the NIR spectrometer. In yet other instances, the method 900 further includes comparing an obtained absorption spectrum against a reference spectrum to determine an authenticity of a beverage sample analyzed via the method 900. In certain instances, the method 900 further includes comparing an obtained absorption spectrum against a reference spectrum to determine whether a beverage line is appropriately producing a beverage.

In some instances, the one or more heat exchangers may provide substantially stable temperature control to help the apparatus of the method 900 provide accurate measurements (e.g., absorption spectra, density values). For instance, when multiple samples are analyzed by the apparatus at different times, a temperature variation of the beverage samples provided to the apparatus 200 at the different times may be within about 0 °C to about 1 °C, or about 0 °C to about 0.1 °C, or about 0.001 °C to about 0.05 °C, or about 0.001 °C to about 0.01 °C. In certain instances, the temperature variation of the beverage samples provided to the apparatus 200 at the different times may be within 0 °C to 1 °C, or 0 °C to 0.1 °C, or 0.001 °C to 0.05 °C, or 0.001 °C to 0.01 °C.

In certain cases, the one or more heat exchangers of the method 900 may be configured or designed to heat or cool the beverage sample within a defined range or a defined tolerance relative to a target temperature. For example, a first heat exchanger of the one or more heat exchangers may be configured or designed to heat or cool a sample within about 1 °C to about 10 °C of a target temperature, a second heat exchanger of the one or more heat exchangers may be configured or designed to heat or cool a sample within about 0 °C to about 1 °C of a target temperature, and a third heat exchanger of the one or more heat exchangers may be configured or designed to heat or cool a sample within about 0 °C to about 0.1 °C of a target temperature. As an additional example, the first heat exchanger may be configured or designed to heat or cool a sample within about 0.5 °C to about 2 °C of the target temperature, the second heat exchanger may be configured or designed to heat or cool the sample within about 0 °C to about 0.75 °C of the target temperature, and the third heat exchanger may be configured or designed to heat or cool a sample within about 0 °C to about 0.01 °C of the target temperature. As yet another example, the first heat exchanger may be configured or designed to heat or cool a sample to within about 0 °C to about 1.5 °C of a target temperature, the second heat exchanger may be configured or designed to heat or cool a sample within about 0 °C to about 0.5 °C of a target temperature, and the third heat exchanger may be configured or designed to heat or cool a sample within about 0 °C to about 0.001 °C of the target temperature.

In further instances, the first heat exchanger may be configured or designed to heat or cool a sample within 1 °C to 10 °C of a target temperature, the second heat exchanger may be configured or designed to heat or cool a sample within 0 °C to 1 °C of a target temperature, and the third heat exchanger may be configured or designed to heat or cool a sample within 0 °C to 0.1 °C of a target temperature. In other instances, the first heat exchanger may be configured or designed to heat or cool a sample within 0.5 °C to 2 °C of the target temperature, the second heat exchanger may be configured or designed to heat or cool the sample within 0 °C to 0.75 °C of the target temperature, and the third heat exchanger may be configured or designed to heat or cool a sample within 0 °C to 0.01 °C of the target temperature. In yet other instances, the first heat exchanger may be configured or designed to heat or cool a sample to within 0 °C to 1.5 °C of a target temperature, the second heat exchanger may be configured or designed to heat or cool a sample within 0 °C to 0.5 °C of a target temperature, and the third heat exchanger may be configured or designed to heat or cool a sample within 0 °C to 0.001 °C of the target temperature.

In some instances, the one or more heat exchangers of the method 900 may be provided with a first heat exchanger that is configured or designed to heat or cool a beverage sample to within about 3 °C, or within about 2.5 °C, or within about 2 °C, or within about 1.9 °C, or within about 1.8 °C, or within about 1.7 °C, or within about 1.6 °C, or within about 1.5 °C, or within about 1.4 °C, or within about 1.3 °C, or within about 1.2 °C, or within about 1.1 °C, or within about 1 °C of a target temperature. In other instances, the one or more heat exchangers of the method 900 may be provided with a first heat exchanger that is configured or designed to heat or cool a beverage sample to within 3 °C, or within 2.5 °C, or within 2 °C, or within 1.9 °C, or within 1.8 °C, or within 1.7 °C, or within 1.6 °C, or within 1.5 °C, or within 1.4 °C, or within 1.3 °C, or within 1.2 °C, or within 1.1 °C, or within 1 °C of a target temperature.

In some instances, the one or more heat exchangers of the method 900 may be provided with a second heat exchanger that is configured or designed to heat or cool a beverage sample to within about 1.5 °C, or within about 1.4 °C, or within about 1.3 °C, or within about 1.2 °C, or within about 1.1 °C, or within about 1 °C, or within about 0.9 °C, or within about 0.8 °C, or within about 0.7 °C, or within about 0.6 °C, or within about 0.5 °C, or within about 0.4 °C, or within about 0.3 °C, or within about 0.2 °C, or within about 0.1 °C of a target temperature. In other instances, the one or more heat exchangers of the method 900 may be provided with a second heat exchanger that is configured or designed to heat or cool a beverage sample to within 1.5 °C, or within 1.4 °C, or within 1.3 °C, or within 1.2 °C, or within 1.1 °C, or within 1 °C, or within 0.9 °C, or within 0.8 °C, or within 0.7 °C, or within 0.6 °C, or within 0.5 °C, or within 0.4 °C, or within 0.3 °C, or within 0.2 °C, or within 0.1 °C of a target temperature.

In some instances, the one or more heat exchangers of the method 900 may be provided with a third heat exchanger that is configured or designed to heat or cool a beverage sample to within about 0.5 °C, or within about 0.4 °C, or within about 0.3 °C, or within about 0.2 °C, or within about 0.1 °C, or within about 0.05 °C, or within about 0.04 °C, or within about 0.03 °C, or within about 0.02 °C, or within about 0.01 °C, or within about 0 °C of a target temperature. In other instances, the one or more heat exchangers of the method 900 may be provided with a third heat exchanger that is configured or designed to heat or cool a beverage sample to within 0.5 °C, or within 0.4 °C, or within 0.3 °C, or within 0.2 °C, or within 0.1 °C, or within 0.05 °C, or within 0.04 °C, or within 0.03 °C, or within 0.02 °C, or within 0.01 °C, or within 0 °C of a target temperature.

It is to be appreciated that the steps of the method 900 may be performed in any order and that any of the steps may be performed more than once. In addition, one or more of the steps of the method 900 may be omitted. Furthermore, the method 900 may be carried out manually or implemented by a control system (e.g., by the control system 150 of FIG. 3). The method 900 may be repeated continuously, repeated from time to time, or performed once.

In a first implementation, an apparatus is provided in the form of a housing including an inlet and an outlet, a fluid conduit defining a fluid flow path between the inlet and the outlet, a light source, a measurement cell in optical communication with the light source, a detector designed to determine an absorption spectrum of the fluid sample, one or more heat exchangers including a first heat exchanger and a second heat exchanger, and a density sensor. The fluid conduit is disposed within the housing. The first heat exchanger and the second heat exchanger are arranged in the fluid flow path and are in thermal communication with the fluid conduit. The apparatus is designed to impart the fluid sample with a target temperature.

In a second implementation, which may include the first implementation, the first heat exchanger and the second heat exchanger are arranged in series, and the first heat exchanger is positioned upstream of the second heat exchanger (relative to a flow of the fluid sample through the fluid flow path).

In a third implementation, which may include either the first or second implementation, the first heat exchanger is designed to impart the fluid sample with a first temperature within 1 °C to 10 °C of the target temperature and the second heat exchanger is designed to impart the fluid sample with a second temperature within 0 °C to 1 °C of the target temperature.

In a fourth implementation, which may include the third implementation, the apparatus may further include a third heat exchanger arranged in series with the first heat exchanger and the second heat exchanger and the third heat exchanger is positioned downstream of the second heat exchanger (relative to a flow of the fluid sample through the fluid flow path). The third heat exchanger is designed to impart the fluid sample with a third temperature within about 0.01 °C of the target temperature.

In a fifth implementation, which may include any of the first through fourth implementations, the detector is provided as part of a NIR spectrometer system.

In a sixth implementation, which may include any of the first through fifth implementations, the apparatus further includes a controller in electronic communication with the detector and the density sensor. The controller analyzes measurements provided from the detector and the density sensor to generate a PLS regression model. The controller also determines if the fluid sample is an authentic beverage upon comparison to a known beverage sample.

In a seventh implementation, which may include any of the first through sixth implementations, the one or more heat exchangers are designed to impart the fluid sample with a temperature of at least 10 °C before the fluid sample is provided to the measurement cell.

In an eighth implementation, which may include any of the first through seventh implementations, the one or more heat exchangers are designed to impart the fluid sample with a temperature of 20 °C to 25 °C before the absorption spectrum is determined by the detector.

In a ninth implementation, which may include any of the first through sixth implementations, the fluid sample may be imparted with a first temperature, and the one or more heat exchangers are designed to impart the fluid sample with a second temperature that is within a predetermined tolerance of the target temperature. The fluid sample is imparted with the second temperature before the fluid sample is provided to the measurement cell. In some instances of the ninth implementation the target temperature is 20 °C. In other instances of the ninth implementation, the target temperature is 25 °C. In various instances of the ninth implementation, the target temperature is 20 °C to 25 °C. In any of the instances of the ninth implementation, the predetermined tolerance is imparted with a value of $\pm$ 1 °C, or $\pm$ 0.5 °C, or $\pm$ 0.1 °C, or $\pm$ 0.01 °C, or $\pm$ 0.001 °C.

In a tenth implementation, which may include any of the first through ninth implementations, the apparatus measures one or more parameters of the fluid sample. The one or more parameters include one or more of a concentration of alcohol, a pH level, a sugar content, a flow rate, a dissolved carbon dioxide concentration, a concentration of one or more aromatics, a protein content, a bitterness value, or a temperature of the fluid sample.

In an eleventh implementation, a method for determining concentration of one or more constituents of a beverage is provided, the method including providing a beverage, where the beverage includes one or more constituents, providing a sample of the beverage to an apparatus including a detector and a density sensor, controlling a temperature of the beverage with one or more heat exchangers associated with the apparatus, measuring a density of the beverage using the density sensor, and determining a concentration of a first constituent of the one or more constituents of the beverage using the detector. It is to be appreciated that the method may utilize any of the apparatuses described with respect to the first to tenth implementations above.

In a twelfth implementation, which may include the eleventh implementation, a controller determines the concentration of the first constituent at least partially based on an absorption spectrum determined by the apparatus.

In a thirteenth implementation, which may include the eleventh or twelfth implementations, the method further includes determining an identity of the beverage utilizing an absorption spectrum created from data obtained from the apparatus.

In a fourteenth implementation, which may include the eleventh through the thirteenth implementations, the first constituent is selected from the group consisting of alcohol, sugar, extract, carbon dioxide, a total carbohydrate content, aromatics, proteins, and bitterness-imparting compounds.

In a fifteenth implementation, which may include any of the eleventh to fourteenth implementations, the first constituent is alcohol or extract.

Further aspects will be disclosed by way of Example:

In Example 1, an apparatus designed to determine one or more parameters of a fluid sample, the apparatus comprising: a housing including an inlet and an outlet; a fluid conduit defining a fluid flow path between the inlet and the outlet, the fluid conduit disposed within the housing; a light source; a measurement cell in optical communication with the light source; a detector designed to determine an absorption spectrum of the fluid sample; one or more heat exchangers including a first heat exchanger and a second heat exchanger, wherein the first heat exchanger and the second heat exchanger are arranged in the fluid flow path and are in thermal communication with the fluid conduit; and a density sensor designed to measure a density of the fluid sample, wherein the apparatus is designed to impart the fluid sample with a target temperature.

In Example 2, the apparatus of Example 1, wherein the first heat exchanger and the second heat exchanger are arranged in series, and the first heat exchanger is positioned upstream of the second heat exchanger relative to a flow of the fluid sample through the fluid flow path.

In Example 3, the apparatus of Example 1 or 2, wherein the first heat exchanger is designed to impart the fluid sample with a first temperature within about 1 °C to about 10 °C of the target temperature, and the second heat exchanger is designed to impart the fluid sample with a second temperature within about 0 °C to about 1 °C of the target temperature.

In Example 4, the apparatus of Example 3 further including a third heat exchanger arranged in series with the first heat exchanger and the second heat exchanger, wherein the third heat exchanger is positioned downstream of the second heat exchanger relative to a flow of the fluid sample through the fluid flow path, and the third heat exchanger is designed to impart the fluid sample with a third temperature within about 0.01 °C of the target temperature.

In Example 5, the apparatus of any one of Examples 1 to 4, wherein the detector is provided as part of a NIR spectrometer system.

In Example 6, the apparatus of any one of Examples 1 to 5, wherein: the apparatus further includes a controller in electronic communication with the detector and the density sensor, the controller analyzes measurements provided from the detector and the density sensor to generate a PLS regression model, the controller determines if the fluid sample is an authentic beverage upon comparison to a known beverage profile.

In Example 7, the apparatus of any one of Examples 1 to 6, wherein the one or more heat exchangers are designed to impart the fluid sample with a temperature of at least 10 °C before the fluid sample is provided to the measurement cell.

In Example 8, the apparatus of any one of Examples 1 to 7, wherein the one or more heat exchangers are designed to impart the fluid sample with a temperature of about 20 °C to about 25 °C before the absorption spectrum is determined by the detector.

In Example 9, the apparatus of any one of Examples 1 to 8, wherein: the fluid sample is imparted with a first temperature, the one or more heat exchangers are designed to impart the fluid sample with a second temperature that is within a predetermined tolerance of the target temperature, the fluid sample is imparted with the second temperature before the fluid sample is provided to the measurement cell.

In Example 10, the apparatus of any one of Examples 1 to 9, wherein the apparatus measures one or more parameters of the fluid sample, and wherein the one or more parameters include one or more of a concentration of alcohol, a pH level, a sugar content, a flow rate, a dissolved carbon dioxide concentration, a concentration of one or more aromatics, a protein content, a bitterness value, or a temperature of the fluid sample.

In Example 11, a system, comprising: a beverage manufacturing line; a measurement device in fluid communication with the beverage manufacturing line, the measurement device comprising: a measurement cell designed to receive a fluid sample, wherein the fluid sample is imparted with a first concentration of extract and a second concentration of alcohol; one or more heat exchangers; an NIR spectrometer including a detector positioned to receive light that passes through the measurement cell; and a controller in communication with the one or more heat exchangers and the NIR spectrometer.

In Example 12, the system of Example 11, wherein the measurement device is positioned in-line with the beverage manufacturing line.

In Example 13, the system of Example 11 or 12, wherein: the controller is housed within the measurement device, the measurement device provides measurements of one or more parameters to the controller, the controller determines a PLS regression model utilizing the measurements.

In Example 14, the system of any one of Examples 11 to 13, wherein the controller determines a third concentration of a first component of the fluid sample, and the third concentration is selected from the group consisting of an alcohol content, a sugar content, an extract concentration, a total carbohydrate content, a dissolved carbon dioxide concentration, a concentration of aromatics, a protein content, and an IBU value.

In Example 15, the system of any one of Examples 11 to 14, wherein: a temperature of the fluid sample is controlled before being provided to the NIR spectrometer when the measurement device is positioned at-line in the beverage manufacturing line, the controller utilizes a PLS regression model to compensate for an effect of the temperature of the fluid sample when the measurement device is positioned in-line or on-line in the beverage manufacturing line.

In Example 16, a method for determining concentrations of one or more constituents of a beverage, comprising: providing the beverage, wherein the beverage comprises one or more constituents; providing a sample of the beverage to an apparatus including a detector and a density sensor; controlling a temperature of the beverage with one or more heat exchangers associated with the apparatus; measuring a density of the beverage using the density sensor; and determining a concentration of a first constituent of the one or more constituents of the beverage using the detector.

In Example 17, the method of Example 16, wherein a controller determines the concentration of the first constituent at least partially based on an absorption spectrum determined by the apparatus.

In Example 18, the method of Example 16 or 17 further including determining an identity of the beverage utilizing an absorption spectrum created from data obtained from the apparatus.

In Example 19, the method of any one of Examples 16 to 18, wherein the first constituent is selected from the group consisting of alcohol, sugar, extract, carbon dioxide, a total carbohydrate content, aromatics, proteins, and bitterness-imparting compounds.

In Example 20, the method of Example 19, wherein the first constituent is the alcohol or the extract.

It will be appreciated by those skilled in the art that while the disclosure has been described above in connection with particular instances and examples, the disclosure is not necessarily so limited, and that numerous other instances, examples, uses, modifications, and departures from the instances, examples and uses are intended to be encompassed by the claims attached hereto. The entire disclosure of each patent and publication cited herein is incorporated by reference as if each such patent or publication were individually incorporated by reference herein. Various features and advantages of the disclosure are set forth in the following claims.

**Claims**

1. An apparatus configured to determine one or more parameters of a fluid sample, the apparatus comprising:

   a housing (204) including an inlet (218) and an outlet (223);
   a fluid conduit (242) defining a fluid flow path between the inlet (218) and the outlet (223), the fluid conduit (242) disposed within the housing (204);
   a light source (232);
   a measurement cell (246) in optical communication with the light source (232);
   a detector (250) configured to determine an absorption spectrum of the fluid sample;
   one or more heat exchangers including a first heat exchanger (226a) and a second heat exchanger (226b), wherein the first heat exchanger (226a) and the second heat exchanger (226b) are arranged in the fluid flow path and are in thermal communication with the fluid conduit (242); and
   a density sensor (228) configured to measure a density of the fluid sample,
   wherein the apparatus is configured to impart the fluid sample with a target temperature.

2. The apparatus of claim 1, wherein the first heat exchanger (226a) and the second heat exchanger (226b) are arranged in series, and the first heat exchanger (226a) is positioned upstream of the second heat exchanger (226b) relative to a flow of the fluid sample through the fluid flow path.

3. The apparatus of claims 1 or 2, wherein the first heat exchanger (226a) is configured to impart the fluid sample with a first temperature within 1 °C to 10 °C of the target temperature, and the second heat exchanger (226b) is configured to impart the fluid sample with a second temperature within 0 °C to 1 °C of the target temperature.

4. The apparatus of claim 3 further including a third heat exchanger (226c) arranged in series with the first heat exchanger (226a) and the second heat exchanger (226b), wherein the third heat exchanger (226c) is positioned downstream of the second heat exchanger (226b) relative to a flow of the fluid sample through the fluid flow path, and the third heat exchanger is configured to impart the fluid sample with a third temperature within 0.01 °C of the target temperature.

5. The apparatus of any of claims 1-4, wherein the detector (250) is provided as part of a NIR spectrometer system (234).

6. The apparatus of any of claims 1-5, wherein:

   the apparatus further includes a controller (152) in electronic communication with the detector (250) and the density sensor (228),
   the controller (152) analyzes measurements provided from the detector (250) and the density sensor (228) to generate a PLS regression model,
   the controller (152) determines if the fluid sample is an authentic beverage upon comparison to a known beverage sample.

7. The apparatus of any of claims 1-6, wherein the one or more heat exchangers (226a, 226b) are configured to impart the fluid sample with a temperature of at least 10 °C before the fluid sample is provided to the measurement cell (246).

8. The apparatus of any of claims 1-7, wherein the one or more heat exchangers (226a, 226b) are configured to impart the fluid sample with a temperature of 20 °C to 25 °C before the absorption spectrum is determined by the detector (250).

9. The apparatus of any of claims 1-8, wherein:

the fluid sample is imparted with a first temperature;
the one or more heat exchangers (226a, 226b) are configured to impart the fluid sample with a second temperature that is within a predetermined tolerance of the target temperature,
the fluid sample is imparted with the second temperature before the fluid sample is provided to the measurement cell (246).

10. The apparatus of any of claim 1-9, wherein the apparatus measures one or more parameters of the fluid sample, and wherein the one or more parameters include one or more of a concentration of alcohol, a pH level, a sugar content, a flow rate, a dissolved carbon dioxide concentration, a concentration of one or more aromatics, a protein content, a bitterness value, or a temperature of the fluid sample.

11. A method for determining concentrations of one or more constituents of a beverage, including:

providing the beverage, wherein the beverage includes one or more constituents;
providing a sample (103) of the beverage to an apparatus (100) including a detector (132) and a density sensor (134);
controlling a temperature of the beverage with one or more heat exchangers (104) associated with the apparatus (100);
measuring a density of the beverage using the density sensor (134); and
determining a concentration of a first constituent of the one or more constituents of the beverage using the detector (132).

12. The method of claim 11, wherein a controller (152) determines the concentration of the first constituent at least partially based on an absorption spectrum determined by the apparatus (100).

13. The method of any of claims 11-12 further including determining an identity of the beverage utilizing an absorption spectrum created from data obtained from the apparatus (100).

14. The method of any of claims 11-13, wherein the first constituent is selected from the group consisting of alcohol, sugar, extract, carbon dioxide, a total carbohydrate content, aromatics, proteins, and bitterness-imparting compounds.

15. The method of any of claims 11-14, wherein the first constituent is an alcohol or extract.

FIG. 1A

FIG. 1B

FIG. 2

EP 4 772 860 A1

FIG. 3

FIG. 4

EP 4 772 860 A1

FIG. 5

FIG. 6

FIG. 7

FIG. 8

902 — PROVIDING A BEVERAGE, WHERE THE BEVERAGE INCLUDES ONE OR MORE CONSTITUENTS

904 — PROVIDING A SAMPLE OF THE BEVERAGE TO AN APPARATUS INCLUDING A DETECTOR AND A DENSITY SENSOR

906 — CONTROLLING A TEMPERATURE OF THE SAMPLE WITH ONE OR MORE HEAT EXCHANGERS PROVIDED IN THE APPARATUS

908 — MEASURING A DENSITY OF THE SAMPLE USING A DENSITY SENSOR OF THE APPARATUS

910 — DETERMINING A CONCENTRATION OF A FIRST CONSTITUENT OF ONE OR MORE CONSTITUENTS OF THE SAMPLE USING THE DETECTOR

FIG. 9

**EUROPEAN SEARCH REPORT**

Application Number

EP 26 15 0548

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/158315 A1 (TRYGSTAD W MARCUS [US] ET AL) 21 June 2012 (2012-06-21) | 1-5,7,9, 10 | INV. G01N21/3577 |
| A | * figures 8, 9 * <br> * paragraph [0062] - paragraph [0065] * <br> * paragraph [0084] * | 6,8, 11-15 | G01N33/14 <br> G01N21/359 <br> G01N21/85 |
| | ----- | | |
| Y | CN 202 351 147 U (SHANDONG STARTING PETROLEUM ENVIRONMENTAL PROT G SCIENCE & TECHNOLOGY) 25 July 2012 (2012-07-25) | 1-5,7,9, 10 | |
| A | * figure 1 * | 6,8, 11-15 | |
| | ----- | | |
| A | LACHENMEIER ET AL: "Rapid quality control of spirit drinks and beer using multivariate data analysis of Fourier transform infrared spectra", FOOD CHEMISTRY ELSEVIER LTD., NL, vol. 101, no. 2, 10 September 2006 (2006-09-10), pages 825-832, XP005852329, ISSN: 0308-8146, DOI: 10.1016/J.FOODCHEM.2005.12.032 [retrieved on 2006-09-10] * sections 2.2, 2.3, 3.3, 3.4 * | 11-15 | |
| | ----- | | |

TECHNICAL FIELDS SEARCHED (IPC)

G01N

The present search report has been drawn up for all claims

| Place of search | Date of completion of the search | Examiner |
|---|---|---|
| Berlin | 29 April 2026 | Meister, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 772 860 A1**

## ANNEX TO THE EUROPEAN SEARCH REPORT
## ON EUROPEAN PATENT APPLICATION NO.

EP 26 15 0548

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2026

| Patent document cited in search report | | Publication date | Patent family member(s) | | Publication date |
|---|---|---|---|---|---|
| US 2012158315 | A1 | 21-06-2012 | CA 2762597 | A1 | 21-06-2012 |
| | | | EP 2469267 | A2 | 27-06-2012 |
| | | | US 2012158315 | A1 | 21-06-2012 |
| CN 202351147 | U | 25-07-2012 | NONE | | |

EPO FORM P0459

For more details about this annex : see Official Journal of the European Patent Office, No. 12/82

34

**EP 4 772 860 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63742734